# EUROPEAN PATENT APPLICATION

(11) **EP 4 629 255 A2**
(43) Date of publication of application: **08.10.2025**
(21) Application number: 25305326.8
(22) Date of filing: 12.03.2025
(51) Int. Cl.: G16H 10/60, G16H 50/20, G16H 50/30

(54) **DEVICE AND METHOD FOR PERSONALIZED HEALTH MANAGEMENT**

(30) Priority: 12.03.2024 US 202463564113 P
(71) Applicant: Hopital Americain de Paris, 92200 Neuilly-sur-Seine (FR)
(72) Inventor: GALLIX, Benoit, 92200 Neuilly-sur-Seine (FR); SIGAL, Robert, 92200 Neuilly-sur-Seine (FR)
(74) Representative: Icosa

(57) **Abstract**

The present invention relates to a device (100) and associated computer-implemented method (400) for obtaining a trained model for prediction of at least one risk score of a user for developing an abnormality with respect to a health status of the user, and to a device (200) and associated method (500) for assisting the user by providing at least one health-related instruction for improving a health status of the user, comprising using a trained model for prediction obtained by the device (100).

## Description

### FIELD OF INVENTION

The present invention relates to the field of digital health technology, in particular, the invention relates to a device and method for obtaining a trained model for prediction of at least one risk score of a user for developing an abnormality with respect to a health status of the user, and to a device and method for assisting the user by providing at least one health-related instruction for improving a health status of the user.

### BACKGROUND OF INVENTION

Prevention is neglected in the French healthcare system, as it is in most developed countries. Citizens are aware of the importance of regular check-ups. In 2003, a French study analyzing the reasons for consultations showed that requests for preventive medicine were the most common reason for consultations - accounting for 11% of the reasons for consultations with general practitioners (GPs). In France, total spending on prevention is estimated at around €15 billion, or less than €230 per inhabitant. Prevention accounts for only 5% of total healthcare spending, which will exceed €320 billion by 2022, or €4,600 per capita.

Prevention is one of the major challenges facing a health policy that is still too focused on curative care. The French plan "Priorité Prévention", introduced in 2018, aims to improve the health of the population through prevention. But concrete actions have been slow to emerge. There is an urgent need to respond to the new health challenges: the increase in chronic diseases and a healthy life expectancy that has not improved significantly despite the increase in health spending. Life expectancy around the world decreased in 2020 due to COVID-19. Most peer countries rebounded by 2021, while the U.S. continued to decline.

If the overall goal is to increase lifespan, it is necessary to individualize lifespan with healthy lifespan. The goal is not necessarily to live to a very old age, but to live longer in good health. This can only be achieved through a preventive approach. A comparison of health care expenditures and life expectancy within the OECD shows that health care expenditures, which consist mainly of curative care, do not improve healthy life expectancy beyond a certain level of spending.

In 1948, the WHO defined health prevention as "approaches and activities aimed at reducing the likelihood that a disease or disorder will affect an individual, interrupting or slowing the progress of the disorder or reducing disability". Three levels of prevention were distinguished: primary, secondary, and tertiary, each linked to successive states of disease.

Primary prevention consists of reducing the number of new cases of a disease over a given period and in a given population. This is known as the incidence of the disease. The goal is to protect the population from the risks to which it is exposed.

Secondary prevention essentially aims to reduce the prevalence of a disease. It concerns people who have developed risk factors or who show preclinical signs of a pathology, but without obvious symptoms. The aim is therefore to detect or diagnose a disease at an early stage. To be effective, screening must target an at-risk population. Those at risk are offered regular check-ups to help detect a developing disease as early as possible.

The goal of tertiary prevention is to reduce the prevalence of recurrence, minimize complications, and limit the sequelae of a disease that has already been diagnosed and treated.

Digital transformation is affecting all sectors, from industry to services. Medicine is no exception to this trend, but is still lagging behind, even though the COVID-19 pandemic has accelerated this transformation.

Increasingly, patients demand direct, personal access to digital health. In the U.S., as in France, accessing healthcare is increasingly done online. A recent study in the US found: 50% of searches for doctors, 40% of searches for hospitals and care facilities, and 75% of appointments are now made online.

As medical technology continues to advance, artificial intelligence (AI) is playing an increasingly important role in the early detection and prevention of disease. AI-powered systems are now being used to detect diseases earlier and more accurately.

The emerging digital twin technology, which has been used primarily by industrial and engineering companies, is becoming a transformative force in healthcare systems. Digital twins will optimize prevention by integrating real-time data, advanced analytics, and virtual simulations. Predictive analytics and preventative interventions are facilitated by digital twin technology, which allows analyzing individual patient data.

Initial studies have shown the value of digital twins for prevention and public health initiatives:
- Modeling and simulating the spread of infectious diseases during the COVID-19 epidemic. These models help epidemiologists and public health officials predict how the disease might spread and design preventive measures,
- Modeling public health based on consumed data,
- Assessing cardiovascular risk factors by monitoring cardiovascular risk factors such as blood pressure, cholesterol and blood sugar levels,
- Creating control arms in clinical trials,
- Improving colorectal cancer screening with fecal tests,
- Early cancer diagnosis,
- Predicting blood glucose levels through individualized analysis of diet and activity.

But this is very preliminary work, and no team currently seems to be working on using the digital twin to monitor and prevent personal health.

In today's healthcare landscape, there's a growing need for simple and user-friendly tools dedicated to helping users manage their health proactively. Currently, there's a gap in the availability of digital platforms specifically designed for prevention and personal health tracking. In particular, there is a lack in tools that are personalized to the user's actual health status and overall well-being and that are able to provide directly to the user a comprehensive holistic picture of his health.

Indeed, providing a complete picture of the user's health is a challenging task primarily due to the extensive amount of information required and the complexity of processing it effectively. Furthermore, making sense of this wealth of information and providing to the user personalized recommendations poses significant challenges. It requires striking a balance between analyzing data and presenting it in a way that's easy to understand and act upon.

Therefore, there is a need to develop a tool that effectively translates complex health data into actionable insights without overwhelming users. This involves not only technological innovation but also a shift in how we approach healthcare and health management. Despite the challenges ahead, the goal remains clear: to create a user-friendly tool that puts users in control of their health journey.

The present invention falls within this context.

### SUMMARY

This invention thus relates to a device for obtaining a trained model for prediction of at least one risk score of a user for developing an abnormality with respect to a health status of the user, wherein the device comprises:
- at least one input configured to receive: at least one user data, measured and/or obtained from said user, and at least one reference data, both said user data and said reference data being related to said abnormality;
- at least one processor configured to:
   ∘ generate a training dataset using said at least one user data and said at least one reference data;
   ∘ obtain said trained model for prediction by feeding said training dataset to a first model, wherein said trained model for prediction is configured to receive as input at least one current user data and provide as output said at least one risk score of the user for developing said abnormality;
- at least one output configured to provide said trained model for prediction.

Advantageously, he trained model for prediction uniquely personalizes to each user by leveraging specific data relevant to their health profile, thereby enhancing prediction accuracy. This model processes a wide array of diverse and heterogeneous data types-ranging from biometric data to environmental exposures-tailored specifically to individual user characteristics. Such comprehensive data integration enables the model to produce highly precise risk assessments that are specific to the individual. This capability not only allows for the early detection of potential health abnormalities but also supports targeted preventive interventions and enhances the effectiveness of clinical decision support systems by providing precise, data-driven insights.

Furthermore, the machine learning model is designed to adapt and improve over time. It achieves this by continuously learning from an ongoing stream of new user data, thereby refining its predictive accuracy. This dynamic adaptation ensures that the model stays relevant and accurate in the long-term, maintaining reliable risk assessments as new health data becomes available. This feature is critical for applications in dynamic health monitoring systems where user conditions may evolve, requiring the model to adjust its predictions based on the latest data.

According to other advantageous aspects of the invention, the device for obtaining a trained model for prediction comprises one or more of the features described in the following embodiments, taken alone or in any possible combination.

According to one embodiment, the user data is obtained from at least one of a medical database, using a wearable sensor, during a clinical examination, and with a diagnostic device.

According to one embodiment, the reference data is obtained from knowledge-based database, comprising established findings at least associated to said abnormality.

According to one embodiment, the first model is an Artificial Neural Network (ANN) or a Convolutional Neural Network (CNN).

According to one embodiment, the first model is a multi-input model comprising at least two input layers, wherein each input layer is configured to handle a type of input data.

Advantageously, the first model is a multi-input model designed to handle complex data structures efficiently. This model comprises at least two distinct input layers, each specialized for processing a specific type of input data. This modular architecture allows for tailored preprocessing of different data types, optimizing the extraction of relevant information from each data stream and thereby improving the model's overall predictive accuracy.

According to one embodiment, said type of input data corresponds to at least one of a numerical data, time-series data, a categorical data, a textual data, an image data, an audio data, and a combination thereof.

Each input layer in this model is adept at managing a variety of data types, including but not limited to: numerical data from clinical measurements, time-series data from continuous monitoring devices, categorical data from patient surveys, textual data from clinical notes, image data from diagnostic imaging tools, and audio data from patient interactions, biological and genetics data. This versatility allows the model to integrate and synthesize data across modalities, enhancing its ability to derive comprehensive insights into the user's health status.

According to one embodiment, said at least one processor configured to generate said training dataset by:
- extracting at least one feature from the user data using the reference data,
- determining at least one risk score associated to the user, based on the at least one feature and the reference data,
- providing the training dataset, wherein said training dataset comprises the user data, the reference data, the at least one extracted feature and the at least one determined risk score.

The model's processor is configured to construct a sophisticated training dataset that includes several transformative steps:

Feature Extraction: Utilizes advanced algorithms to extract significant features from the user data, employing the reference data as a benchmark to identify patterns or anomalies indicative of health risks.

Risk Score Determination: Calculates one or more risk scores for the user by analyzing extracted features in conjunction with the reference data. This step involves sophisticated modeling techniques to quantify risk levels based on a combination of current user data and historical health data.

Dataset Compilation: Compiles the training dataset, which comprises the original user data, the utilized reference data, the extracted features, and the newly determined risk scores. This dataset is then ready for use in training the machine learning model, ensuring that it is well-equipped to make accurate and personalized health risk assessments.

This invention further relates to a device for assisting the user by providing at least one health-related instruction for improving a health status of the user, wherein the device for assisting the user comprises:
- at least one input configured to receive at least one current user data;
- at least one processor configured to:
   ∘ calculate at least one risk score of the user for developing said abnormality using a trained model for prediction obtained by the device for obtaining a trained model for prediction according to any of the previous embodiments;
   ∘ provide as input to a trained second model said at least one risk score to obtain as output said at least one health-related instruction, said trained second model being previously trained using a database comprising at least risk scores and associated instructions;
at least one output configured to provide said at least one health-related instruction.

According to one embodiment, within the device for assisting the user, the health-related instruction is at least one of the following: a proposition to undergo a medical examination, an invitation to schedule an appointment with a healthcare professional, an advice to modify a lifestyle habit, an instruction to trigger an alert, an instruction to control a peripheral device, and an instruction to establish a communication with another user.

According to one embodiment, within the device for assisting the user, a command is associated with said health-related instruction.

According to one embodiment, the device for assisting the user further comprises at least one graphical interface to display a virtual model of the user, preferably obtained using a biostatistical model.

According to one embodiment, within the device for assisting the user, the risk score is used at least to display, in the at least one graphical interface, a health status of at least one organ of the user on the virtual model of the user.

According to one embodiment, within the device for assisting the user, the health-related instruction is displayed to the user on the graphical interface, and the command associated with the health-related instruction consists in at least one of the following:
- sending a notification to the user related to a health status of the user; and/or
- displaying a report to the user related to a health status of the user; and/or
- sending a control signal to at least one peripheral device; and/or
- sending a control signal to at least one receiving interface.

This invention further relates to a computer implemented method for obtaining a trained model for prediction of at least one risk score of a user for developing an abnormality with respect to a health status of the user, said method comprising:
- receiving at least one user data, measured and/or obtained from said user, and at least one reference data, both said user data and said reference data being related to said abnormality;
- generating a training dataset using said at least one user data and said at least one reference data;
- obtaining said trained model for prediction by feeding said training dataset to a first model, wherein said trained model for prediction is configured to receive as input at least one current user data and provide as output said at least one risk score of the user for developing said abnormality;
- providing said trained model for prediction.

This invention further relates to a computer implemented method for assisting the user by providing at least one health-related instruction for improving a health status of the user, said method for assisting comprising:
- receiving at least one current user data;
- calculating at least one risk score of the user for developing said abnormality using a trained model for prediction obtained by the device for obtaining a trained model for prediction according to any of the previous embodiments;
- providing as input to a trained second model said at least one risk score to obtain as output said at least one health-related instruction, said trained second model being previously trained using a database comprising at least risk scores and associated instructions;
- providing said at least one health-related instruction.

The disclosure further relates to a computer program comprising software code adapted to perform a method for obtaining a trained model for prediction and/or a method for assisting the user compliant with any of the above execution modes when the program is executed by a processor.

The disclosure further relates to a computer program product comprising instructions which, when the program is executed by a computer, cause the computer to carry out any of the previously mentioned computer implemented methods.

The disclosure further relates to a computer-readable storage medium comprising instructions which, when executed by a computer, cause the computer to carry out any of the previously mentioned computer implemented methods.

The computer-readable storage medium is a non-transitory program storage device, readable by a computer, tangibly embodying a program of instructions executable by the computer to perform any of the previously mentioned computer implemented methods, compliant with the present disclosure.

Such a non-transitory program storage device can be, without limitation, an electronic, magnetic, optical, electromagnetic, infrared, or semiconductor device, or any suitable combination of the foregoing. It is to be appreciated that the following, while providing more specific examples, is merely an illustrative and not exhaustive listing as readily appreciated by one of ordinary skill in the art: a portable computer diskette, a hard disk, a ROM, an EPROM (Erasable Programmable ROM) or a Flash memory, a portable CD-ROM (Compact-Disc ROM).

This invention enables cross-disciplinary integration of all a patient's medical data (i.e. user data) with a view to prevention. It integrates cutting-edge technologies within an applied framework not previously available in the field of digital health. Advantageously, the present invention combines artificial intelligence models (AI), and predictive analytics, and optionally virtual twins (i.e. virtual model), to model and predict the individual health journey. This is a significant advance that goes beyond simple data collection, offering in-depth, personalized analysis that can inform tailored preventive interventions.

The invention not only monitors the patient's (i.e. the user's) current state of health, but also uses advanced predictive modeling to anticipate future risks. It integrates physiological, behavioral and environmental data into a digital platform that uses AI to assess and recommend personalized prevention strategies. Risk (i.e., risk scores) visualization in the form of, for example interactive radar graph, is also advantageous as it provides an intuitive understanding of information that might not be immediately obvious to an expert in the field. In addition, the emphasis on simplified and transparent communication between patients and caregivers represents a significant advance on existing practices.

The invention may be of interest in diverse areas such as:
- Personalized health: Use in hospitals and clinics to provide personalized care based on each patient's specific risks.
- Prevention: Integration into corporate wellness programs to improve overall employee health and reduce costs associated with illness and absence.
- Clinical research: Use to collect data and create cohorts for clinical trials, improving participant selection and results monitoring.
- Health insurance: Application to develop insurance models based on more accurate risk assessments.
- Pharmaceutical development: Identification of new markets for preventive drugs based on predictive risk data.
- Wearable technologies: Integration with wearable devices for real-time health monitoring and data collection.
- Public health: Use by public health authorities to monitor and respond to health trends at population level.

### DEFINITIONS

In the present invention, the following terms have the following meanings:

The terms "adapted" and "configured" are used in the present disclosure as broadly encompassing initial configuration, later adaptation or complementation of the present device, or any combination thereof alike, whether effected through material or software means (including firmware).

The term "processor" should not be construed to be restricted to hardware capable of executing software, and refers in a general way to a processing device, which can for example include a computer, a microprocessor, an integrated circuit, or a programmable logic device (PLD). The processor may also encompass one or more Graphics Processing Units (GPU), whether exploited for computer graphics and image processing or other functions. Additionally, the instructions and/or data enabling to perform associated and/or resulting functionalities may be stored on any processor-readable medium such as, e.g., an integrated circuit, a hard disk, a CD (Compact Disc), an optical disc such as a DVD (Digital Versatile Disc), a RAM (Random-Access Memory) or a ROM (Read-Only Memory). Instructions may be notably stored in hardware, software, firmware or in any combination thereof.

"Machine learning (ML)" designates in a traditional way computer algorithms improving automatically through experience, on the ground of training data enabling to adjust parameters of computer models through gap reductions between expected outputs extracted from the training data and evaluated outputs computed by the computer models.

A "hyper-parameter" presently means a parameter used to carry out an upstream control of a model construction, such as a remembering-forgetting balance in sample selection or a width of a time window, by contrast with a parameter of a model itself, which depends on specific situations. In ML applications, hyper-parameters are used to control the learning process.

"Datasets" are collections of data used to build an ML mathematical model, so as to make data-driven predictions or decisions. In "supervised learning" (i.e. inferring functions from known input-output examples in the form of labelled training data), three types of ML datasets (also designated as ML sets) are typically dedicated to three respective kinds of tasks: "training", i.e. fitting the parameters, "validation", i.e. tuning ML hyperparameters (which are parameters used to control the learning process), and "testing", i.e. checking independently of a training dataset exploited for building a mathematical model that the latter model provides satisfying results.

A "neural network (NN)" designates a category of ML comprising nodes (called "neurons"), and connections between neurons modeled by "weights". For each neuron, an output is given in function of an input or a set of inputs by an "activation function". Neurons are generally organized into multiple "layers", so that neurons of one layer connect only to neurons of the immediately preceding and immediately following layers.

The above ML definitions are compliant with their usual meaning and can be completed with numerous associated features and properties, and definitions of related numerical objects, well known to a person skilled in the ML field. Additional terms will be defined, specified or commented wherever useful throughout the following description.

### BRIEF DESCRIPTION OF THE DRAWINGS

The present disclosure will be better understood, and other specific features and advantages will emerge upon reading the following description of particular and non-restrictive illustrative embodiments, the description making reference to the annexed drawings wherein:

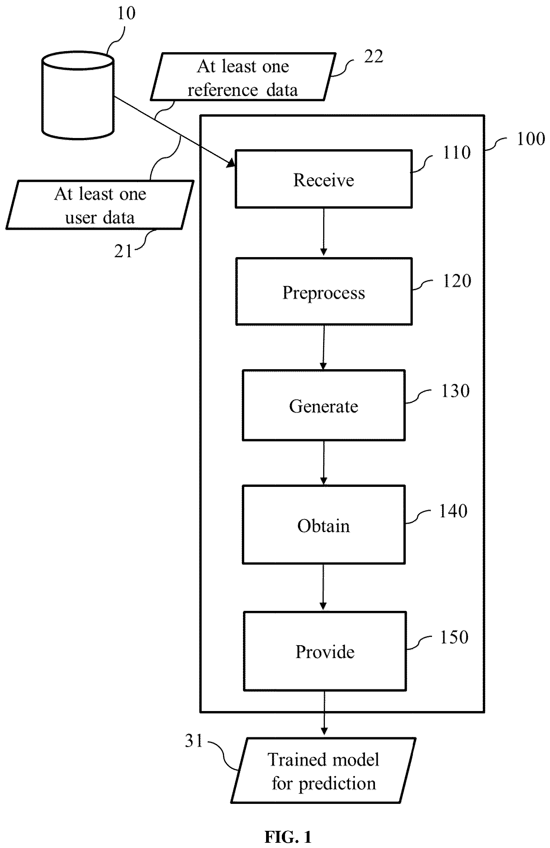

**Figure 1** is a block diagram representing schematically a particular mode of a device for obtaining a trained model for prediction of at least one risk score of a user for developing an abnormality with respect to a health status of the user, compliant with the present disclosure;

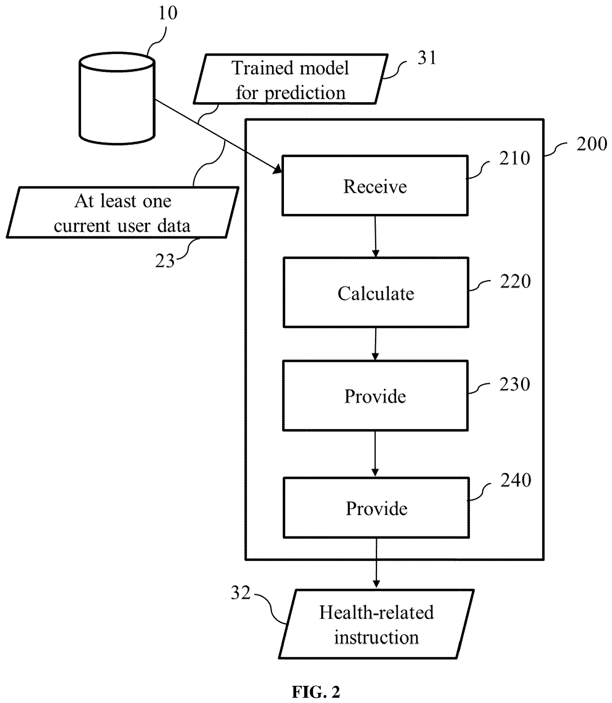

**Figure 2** is a flow chart showing successive steps of the computer-implemented method executed by the device of figure 1;

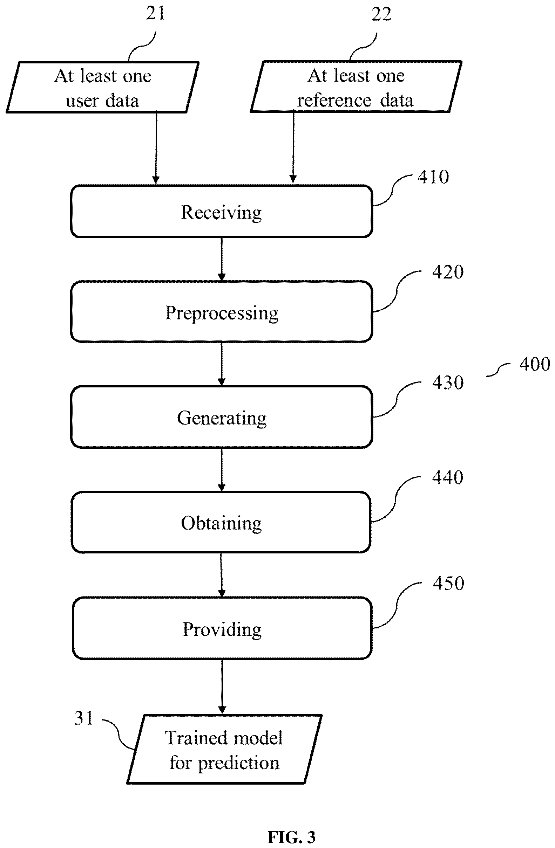

**Figure 3** is a block diagram representing schematically a particular mode of a device for assisting the user by providing at least one health-related instruction for improving a health status of the user using a trained model for prediction obtained using the device of figure 1, compliant with the present disclosure;

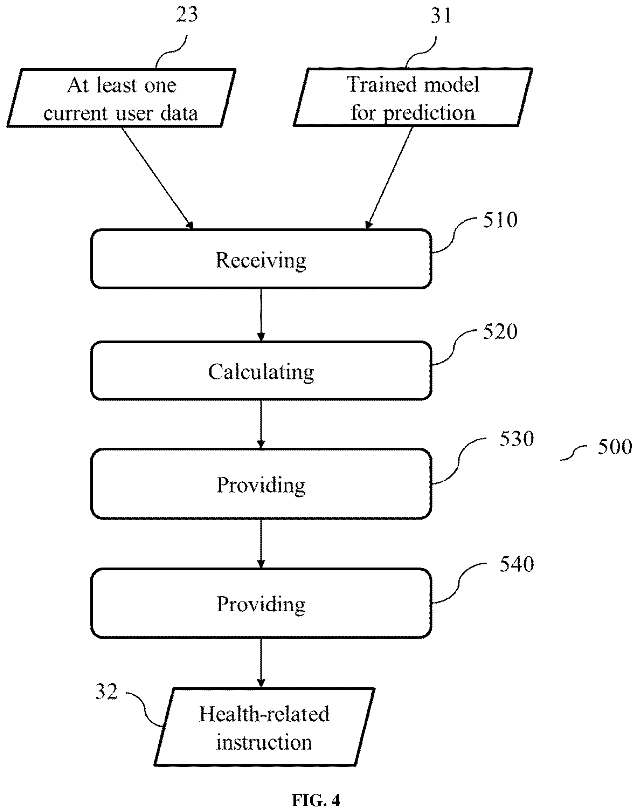

**Figure 4** is a flow chart showing successive steps of the computer-implemented method executed by the device of figure 3;

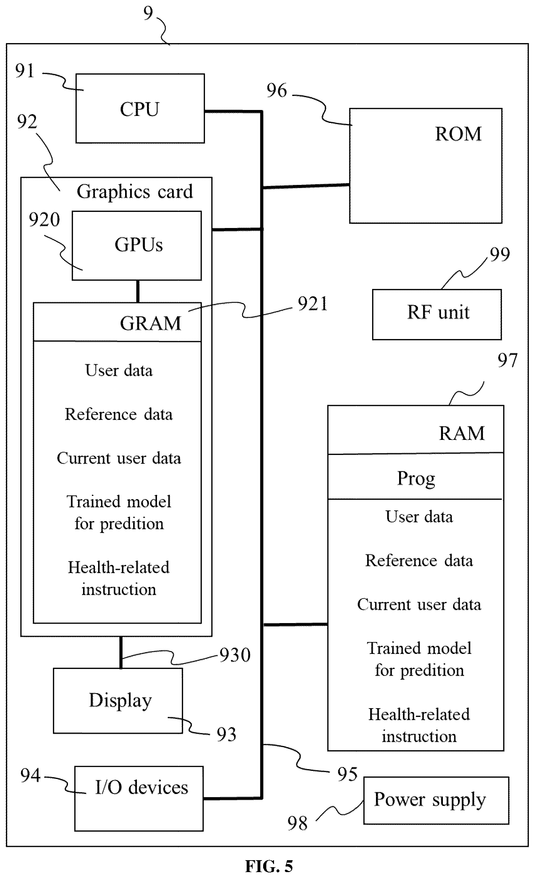

**Figure 5** diagrammatically shows an apparatus integrating the functions of the devices of figures 1 and 3;

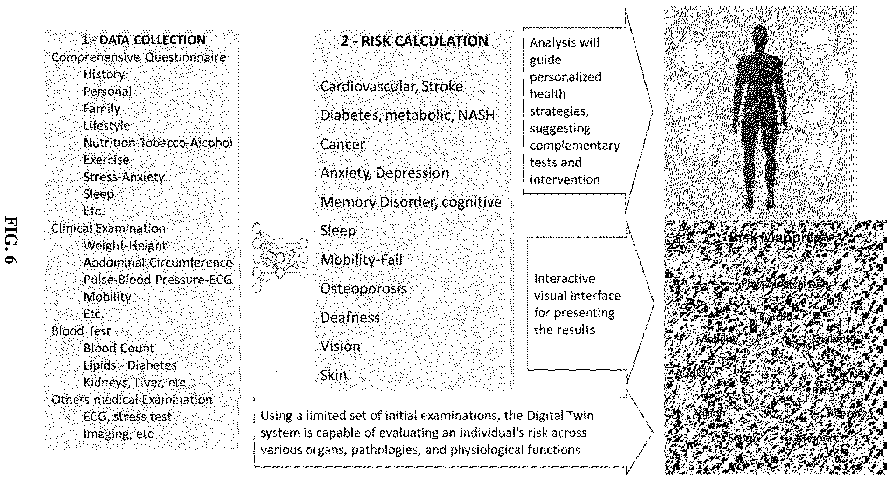

**Figure 6** illustrates the three-stage process for personalized health risk assessment, wherein Stage 1 involves comprehensive data collection through questionnaires and clinical exams, Stage 2 calculates individual health risks for a range of conditions from cardiovascular to cognitive health and the final stage presents the results via an interactive interface, mapping both chronological and physiological health risks across various domains;

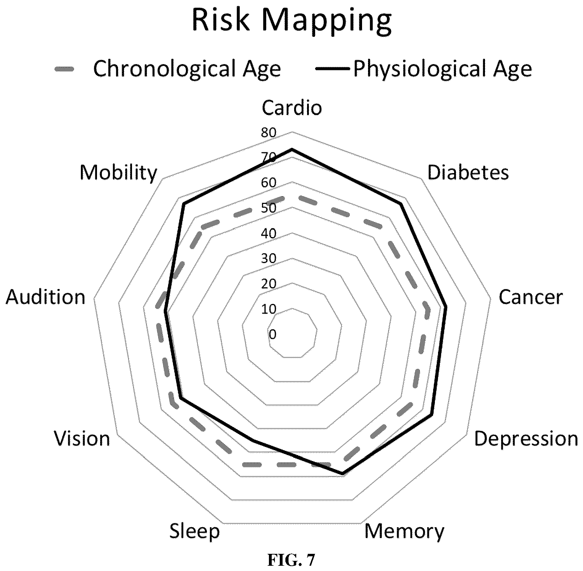

**Figure 7** illustrates a comparative analysis of an individual's chronological age versus their physiological age across various health domains including Cardiovascular Health, Diabetes, Cancer, Depression, Memory, Sleep, Vision, Audition, and Mobility;

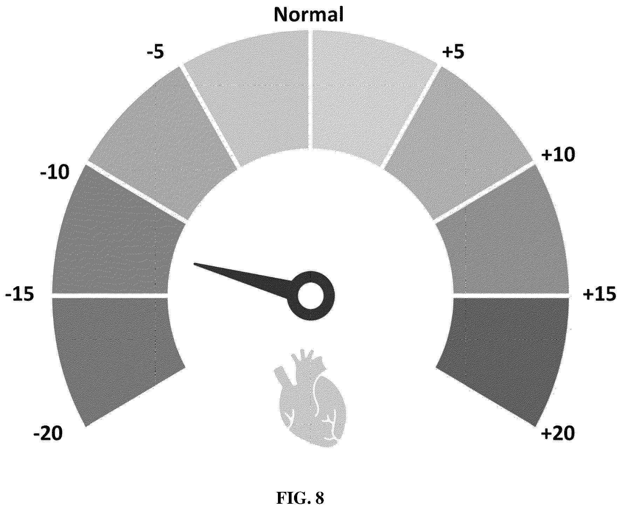

**Figure 8** is a Health Risk Age Gauge for one organ or disease domain that quantifies the relative age-related risk for a specific organ or disease compared to the average population; The dial measures the deviation in years from the normal risk age, with "Normal" positioned at the center; Values to the left, indicated by negative numbers, suggest a risk age lower than average, potentially signifying better health or reduced risk; Conversely, positive values to the right denote a higher risk age, implying increased health risks or conditions advancing more rapidly than typical; The scale extends from - 20 to +20 years, offering an immediate visual assessment of how an individual's risk compares to the norm, aiding in personalized health management discussions;

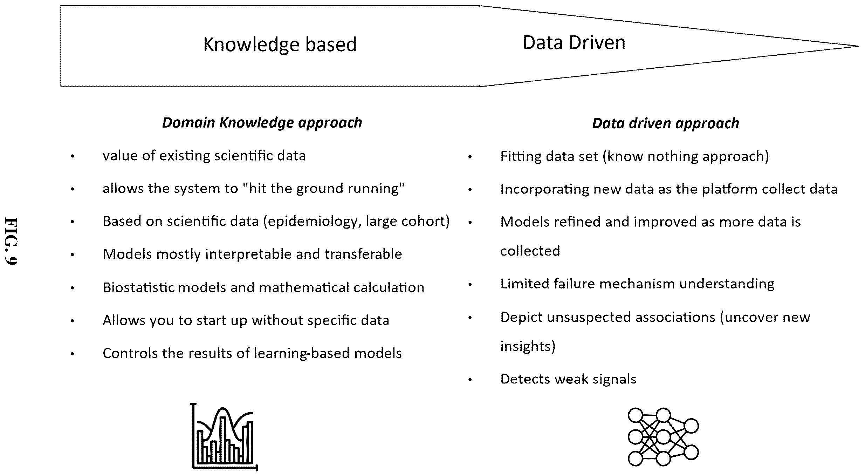

**Figure 9** illustrates the synergistic use of both Knowledge-Based and Data-Driven approaches within the platform; Initially, the platform utilizes a Knowledge-Based approach, leveraging established scientific data to quickly operationalize ("hit the ground running") and ensure models are interpretable and reliable; Concurrently, it adopts a Data-Driven strategy to progressively refine these models by learning from continuously accumulated data, enhancing predictive accuracy and uncovering new insights; This dual approach allows for immediate effective deployment and ongoing improvement, optimizing the system's performance across various domains;

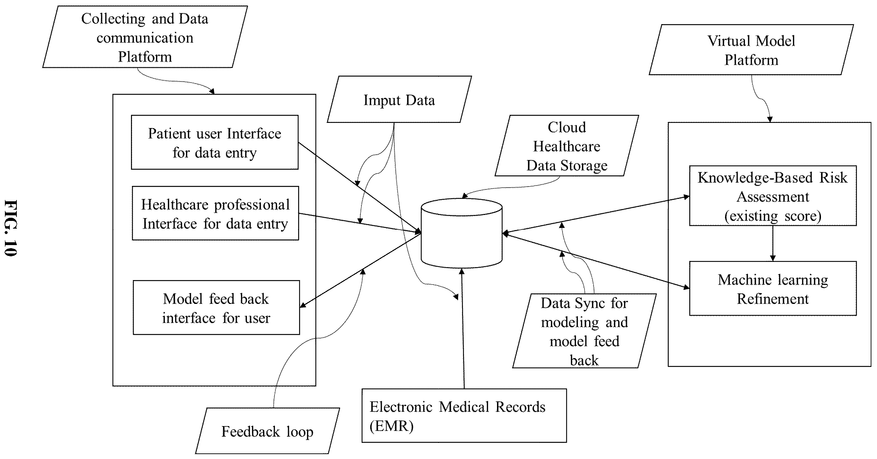

**Figure 10** illustrates an advanced healthcare management system that integrates a Collecting Data Platform with a Virtual Model Platform; Patients interact with the system via the Collecting Data Platform, entering health data and accessing results; This data flows through the Electronic Medical Records (EMR) into the Cloud Healthcare Data Storage (Cloud), where it is securely stored and aggregated; The Virtual Model Platform then uses this data to perform advanced analyses and simulations, providing predictive health insights; Results are fed back to patients through the Collecting Data Platform, enhancing patient engagement and personalized health management;

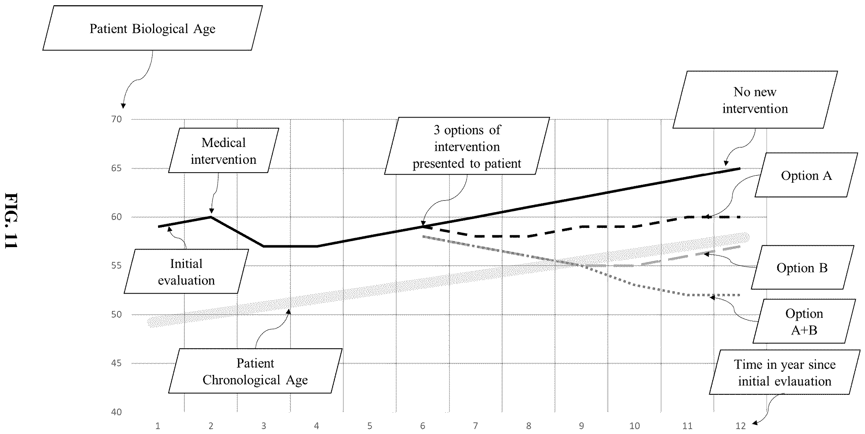

**Figure 11** is a graphic evaluating the Impact of Health Interventions on Biological Age; This chart is strategically designed to provide patients with insightful feedback on how both pharmaceutical and non-pharmaceutical interventions influence their biological age and, ultimately, their longevity; The different lines are computed by the Virtual Model Platform; The solid black line shows the baseline biological age progression without new intervention in the future intervention. After 2 years of follow up the patient was treated with medication to improve risk (medical intervention) and the computed curve improve; Each subsequent line demonstrates the effect of different interventions, as calculated by the platform: Solid Black Line: No new intervention, natural progression of biological age; Dashed Line (Option A): Depicts the impact of engaging in regular exercise; Dotted Line (Option B): Shows the of rigourous diet and weight loss; Dash-Dot Line (Option A+B): Represents the combined benefits of both exercise and diet; The chronological age of the patient is indicated by the light gray line for reference; This visualization helps patients and healthcare providers understand how specific interventions can modify the aging process, facilitating informed decisions for health management and tracking the impact.

On the figures, the drawings are not to scale, and identical or similar elements are designated by the same references.

### ILLUSTRATIVE EMBODIMENTS

The present description illustrates the principles of the present disclosure. It will thus be appreciated that those skilled in the art will be able to devise various arrangements that, although not explicitly described or shown herein, embody the principles of the disclosure and are included within its scope.

All examples and conditional language recited herein are intended for educational purposes to aid the reader in understanding the principles of the disclosure and the concepts contributed by the inventor to furthering the art, and are to be construed as being without limitation to such specifically recited examples and conditions.

Moreover, all statements herein reciting principles, aspects, and embodiments of the disclosure, as well as specific examples thereof, are intended to encompass both structural and functional equivalents thereof. Additionally, it is intended that such equivalents include both currently known equivalents as well as equivalents developed in the future, i.e., any elements developed that perform the same function, regardless of structure.

Thus, for example, it will be appreciated by those skilled in the art that the block diagrams presented herein may represent conceptual views of illustrative circuitry embodying the principles of the disclosure. Similarly, it will be appreciated that any flow charts, flow diagrams, and the like represent various processes which may be substantially represented in computer readable media and so executed by a computer or processor, whether or not such computer or processor is explicitly shown.

The functions of the various elements shown in the figures may be provided through the use of dedicated hardware as well as hardware capable of executing software in association with appropriate software. When provided by a processor, the functions may be provided by a single dedicated processor, a single shared processor, or a plurality of individual processors, some of which may be shared.

It should be understood that the elements shown in the figures may be implemented in various forms of hardware, software or combinations thereof. Preferably, these elements are implemented in a combination of hardware and software on one or more appropriately programmed general-purpose devices, which may include a processor, memory and input/output interfaces.

The present disclosure will be described in reference to a particular functional embodiment of a device 100 for obtaining a trained model for prediction of at least one risk score of a user for developing an abnormality with respect to a health status of the user, as illustrated on **Figure 1****.**

Device 100 may be configured to:
- receive user data 21 related to the anomality, measured and/or obtained from the user, and reference data 22, both the user data and the reference data may be related to the abnormality; and,
- provide as output the trained model for prediction 31.

The device 100 is associated with a device 200 for assisting the user by providing at least one health-related instruction for improving a health status of the user, using the trained model for prediction 31 obtained from the device 100. The device 200 is represented on **Figure 2****,** and will be subsequently described.

Device 200 is configured to receive at least one current user data 23 and output at least one health-related instruction 32.

Though the presently described devices 100 and 200 are versatile and provided with several functions that can be carried out alternatively or in any cumulative way, other implementations within the scope of the present disclosure include devices having only parts of the present functionalities.

Each of the devices 100 and 200 is advantageously an apparatus, or a physical part of an apparatus, designed, configured and/or adapted for performing the mentioned functions and produce the mentioned effects or results. In alternative implementations, any of the device 100 and the device 200 is embodied as a set of apparatus or physical parts of apparatus, whether grouped in a same machine or in different, possibly remote, machines. The device 100 and/or the device 200 may e.g. have functions distributed over a cloud infrastructure and be available to users as a cloud-based service, or have remote functions accessible through an API.

The device 100 and the device 200 for may be integrated in a same apparatus or set of apparatus, and intended to same users. In other implementations, the structure of device 200 may be completely independent of the structure of device 100, and may be provided for other users.

In what follows, the modules are to be understood as functional entities rather than material, physically distinct, components. They can consequently be embodied either as grouped together in a same tangible and concrete component, or distributed into several such components. Also, each of those modules is possibly itself shared between at least two physical components. In addition, the modules are implemented in hardware, software, firmware, or any mixed form thereof as well. They are preferably embodied within at least one processor of the device 100 or the device 200.

Device 100 may comprise a module 110 configured to receive, from one or more local or remote database(s) 10, at least one user data 21, measured and/or obtained from the user, and at least one reference data 22, both the user data 21 and the reference data 22 being related to the abnormality. The database(s) 10 can take the form of storage resources available from any kind of appropriate storage means, which can be notably a RAM or an EEPROM (Electrically-Erasable Programmable Read-Only Memory) such as a Flash memory, possibly within an SSD (Solid-State Disk). Alternatively, the at least one user data 21 and at least one reference data 22 may be received from a communication network. According to an embodiment, some input(s) may be stored in one or more local or remote database(s) 10 and other (s) may be received from a communication network.

According to the invention, the user data 21 refer to a collection of data personal to the user, gathered from various sources. This includes data collected by the healthcare system, data provided directly by the user, and data derived from other external sources. The user data 21 may pertain to a single user or multiple users, such as for instance at least two users. In that case, the user data 21 comprises multiple sets of user data, wherein one set of user data is associated to one user of the plurality of users.

The user data 21 may comprise at least one information or at least two information. In the same manner, each set of data may comprise at least one information or at least two information.

The user data 21 may comprise demographic information (e.g., the user's name, age, sex, height, weight etc.), the health history (e.g., past diseases, current medication, vaccination history), clinical examination results (e.g., biological samples analysis results, imaging data, time series data, genomic or omics data), lifestyles factors (e.g., tobacco use, alcohol consumption, physical activity). Furthermore, the data may reflect aspects of the user's mood, energy levels, sleep patterns, diet and mental health (e.g., stress, anxiety).

The user data 21 may be in various formats (e.g., text, image, audio, video etc.).

The user data 21 may for instance be collected through structured question-and-answer formats (e.g., Yes/No responses) or through patient-reported information via surveys or apps, using wearable sensors (e.g., personal wearable devices like fitness trackers or smartwatches or medical wearable device such as blood pressure monitors or glucose meters), diagnostic devices (MRI, CT scan, X-ray etc.). Additional sources may include social media data, environmental sensors, or data from Internet of Medical Things (IMoT) devices that track daily activity, sleep, or environmental conditions.

The user data 21 comprises at least one information that can be linked to an abnormality. For instance, a single information may deviate from typical results, or multiple information may be correlated to identify such deviations. By monitoring and correlating data from multiple sources, deviations from typical physiological behavior can be detected, potentially indicating physical, physiological, symptomatic, diagnostic, behavioral, or pathological abnormalities that may require further investigation or intervention.

To ensure the privacy and security of the user data 21, secure methods may be implemented. These methods would include the use of encryption, anonymization, and access controls to protect sensitive information. Additionally, secure ETL (Extract, Transform, Load) processes can be employed to efficiently ingest, clean, and standardize data from various sources, ensuring that the user data 21 is accurate, consistent, and securely stored before being processed. This approach safeguards the integrity of the data while maintaining user confidentiality.

The reference data 22 refers to a set of established findings and knowledge used as a baseline for comparison or analysis, particularly in relation to abnormalities. Literature data, particularly on known risk factors for diseases, already exists and may serve as a reliable starting point for obtaining the reference data 22.

The reference data 22 may be obtained from various types of knowledge-based databases, which store information relevant to the abnormality being investigated. Such databases can include relational databases, NoSQL databases, graph databases, semantic databases, document databases (e.g., scientific articles), or knowledge graph databases.

The reference data 22 may take various forms, such as continuous or categorical user data, image data, temporal signal data, text data, or multi-modal data, depending on the type of analysis being conducted.

According to the invention, an abnormality may be physical (e.g. structural anomalies or irregularities in the body's organs, tissues, or systems, such as congenital malformations, tumors, or anatomical variation), physiological (dysfunction or imbalance in bodily functions, such as abnormal heart rhythms, hormonal imbalances, or metabolic disorders), symptomatic (e.g. unusual or atypical signs and symptoms that may indicate underlying health issues, such as persistent fatigue, unexplained weight loss or gain, or recurring pain), diagnostical (abnormal findings in medical tests or diagnostic procedures, including abnormal laboratory test results, imaging abnormalities, or abnormal electrocardiogram (ECG) readings), behavioral (e.g. health-related behaviors that deviate from normal patterns and may indicate underlying health problems, such as excessive alcohol consumption, substance abuse, or unusual eating habits), a pathology or any kind of deviation from the physiological behavior.

Device 100 may further comprise a module 120 for preprocessing the input data (i.e. user data 21, reference data 22). The input data may undergo a preprocessing adapted to the type of data (e.g., numerical, text, image, audio, continuous data, time-frequency data, categorical data). Each type of data may undergo standardization and normalization, to standardize different data formats, units, and/or scales. Data cleaning may be applied to identify and correct inaccuracies, outliers, missing values, and inconsistencies. The input data may further undergo dimensional reduction to reduce redundant or irrelevant data (e.g., using Principal Component Analysis (PCA) for numerical data, word embeddings (BERT, Word2Vec) for text data etc.). Additionally, data enrichment techniques may be used to enhance user data, by generating synthetic data (e.g., generated using stochastic simulation), or by computing derived metrics like Body Mass Index (BMI), SCORE2, and Framingham risk scores from existing user data (e.g., height, weight for BMI, age, sex, smoking status, systolic blood pressure, total cholesterol, High-density lipoprotein cholesterol for SCORE2and Framingham risk).

For numerical data (e.g., obtained from sensors, medical tests, or physiological measurements), preprocessing may involve scaling techniques such as z-score normalization or Min-Max scaling, transformations (e.g., logarithmic or Box-Cox transformations) to reduce skewness, and outlier handling using methods like Interquartile Range (IQR) or Z-score detection.

Categorical data (e.g., nominal data such as blood type, gender, smoking status, ordinal data such as pain severity, cancer stage, survey response) may undergo encoding (e.g., one-hot encoding for nominal variables or ordinal encoding for ranked categories), cardinality reduction (e.g., binning low-frequency categories), and missing value imputation using statistical methods or predictive models. To prevent multicollinearity in regression models, dummy variable trap avoidance techniques may be applied.

For image data, preprocessing may include image recognition algorithms to detect patterns, as well as converting medical images into formats suitable for machine learning models.

For time-series data (e.g., ECG or other physiological signals), signal processing techniques may be employed to filter noise. These preprocessing steps ensure that user and reference data are clean, structured, and optimized for further processing, analysis, and model training.

The user data 21 may be reorganized using a Master Patient Index (MPI). The MPI acts as a linking mechanism that helps organize and consolidate patient data from various sources (such as EHRs, lab results, imaging systems, etc.) under a unique patient identifier. This process ensures that all relevant data for a specific patient is correctly associated, even if it comes from different healthcare systems or departments. The MPI may be constructed through a combination of data matching algorithms, and data linking. Common algorithms used in MPI construction include exact matching, fuzzy matching (Levenshtein, Soundex), probabilistic matching, rule-based matching, clustering algorithm, machine learning-based methods and a combination thereof.

Device 100 may further comprise a module 130 for generating a training dataset using the at least one user data and the at least one reference data.

The training dataset generation may involve several sub-steps including a first sub-step of extraction of at least one feature from the user data 21 using the reference data 22.

Feature extraction may be performed manually by at least one expert (e.g., a doctor or medical researcher). The expert may use the reference data 22 to determine the at least one feature. The expert may apply his domain-specific knowledge, identify relevant patterns, and utilize predefined rules or guidelines, notably available in the reference data 22. The expert may map the user data 21 to standardized concepts or use the reference data 22 to create new features that are relevant to the prediction task. This process ensures that the extracted features are both clinically significant and aligned with established medical practices or literature.

Feature extraction may also be performed automatically using a feature extractor model. A specific feature extractor model may be used for each type of user data 21. The feature extractor model may take as input the reference data 22 for feature extraction. The feature extractor model may also create new features that are relevant to the prediction task. Feature extractor models commonly known in the prior art can be utilized for this task.

Each extracted feature comprises a label (e.g., age, cholesterol level, diet) and the associated data extracted or derived from the user data 21.

Among the extracted features, some may be more relevant than others regarding the prediction task. Thus, dimensionality reduction may be applied (e.g., PCA, feature selection) may be used to remove irrelevant or redundant features.

The training dataset generation may involve a second sub-step of determining at least one risk score associated to the user, using the extracted features and the reference data 22.

Risk-score extraction can be performed through two approaches: manually by at least one expert, such as a doctor or medical researcher, who utilizes their domain knowledge and clinical guidelines, notably available in the reference data 22, or automatically through computational methods leveraging machine learning algorithms, which can process large datasets (e.g., reference data 22) and derive risk scores based on predefined rules or learned patterns from historical data. Machine learning algorithms commonly known in the prior art can be utilized for this task.

For instance, the risk score may comprise a label (e.g., "risk score") and an associated calculated value.

The training dataset may thus comprise the user data 21 (raw data), the reference data 22 received as input, the extracted features and the risk score received as input or calculated.

Device 100 may further comprise a module 140 for obtaining the trained model for prediction by feeding the generated training dataset to a first model (i.e., untrained model architecture).

To process heterogeneous data efficiently, the first model architecture may be a multi-input model designed to handle each type of input data separately (i.e., the input data are heterogeneous data collected from multiple and diverse sources, which may vary in type, format, structure, and nature), then combine their extracted features into a unified representation for downstream analysis.

To achieve this, the multi-input model may comprise distinct input layers, each dedicated to a particular data type or an ensemble of similar data types. These layers process the data independently, ensuring that each data modality is treated according to its unique characteristics.

The training dataset may be divided into multiple subsets (e.g., at least two subsets) based on the nature of the features (e.g. features extracted from text, images, numerical data, etc.), that the model may process through separate input layers. For instance, the numerical features go to one input layer, the text data to a second input layer, and so on.

The input layers may rely on algorithms adapted to the type of feature and that can be trained using the corresponding subsets to be used to extract features of that type.

For numerical data, PCA, ICA, autoencoders, clustering algorithms (e.g., K-Means, DBSCAN) architectures may be implemented.

Categorical data may be processed through embedding layers in deep learning models to transform them into dense vectors of continuous numbers, where the distance between vectors represents the similarity between categories. Alternatively, decision Trees (e.g., XGBoost, LightGBM) or random forests may be used. Feature Selection Algorithms (e.g., LASSO, Recursive Feature Elimination) may be used to identify the most important features by penalizing or eliminating irrelevant ones for both numerical data and categorical data. Additionally, techniques like Deep Feature Synthesis can be used to create new features from raw data for numerical data and categorical data, enhancing the information available for model training. Variational Autoencoders (VAEs) can also be employed for both continuous and categorical data in an unsupervised learning context, enabling the model to capture complex relationships and reduce dimensionality.

Image data may be processed through deep learning models, such as Convolutional Neural Networks (CNNs) or 3D CNNs, which are specifically designed to analyze spatial hierarchies in images. These models can automatically detect and extract relevant features from raw image data, such as identifying key structures or abnormalities. Pre-trained models like ResNet, Inception, and EfficientNet, which have been trained on large datasets like ImageNet, can be fine-tuned to improve performance on medical images, enabling them to extract complex features that are critical for tasks like detecting tumors, calculating calcium scores, or identifying other abnormalities. The extracted features from these models may be converted into numerical data, which can be used for further processing.

Text data, such as clinical notes, patient histories, or doctor's reports, is often unstructured and can be challenging to process directly. Initially, medical data is typically structured during the collection process, but unstructured text data may require advanced techniques to extract features. Natural Language Processing (NLP) and Large Language Models (LLMs) like BERT, GPT, and their derivatives, which are pre-trained on vast corpora, can be fine-tuned for specific healthcare tasks to provide contextually rich representations of the text data. These models can perform tasks such as information extraction, which identifies clinical entities like diseases, medications, or symptoms within the text. Additionally, NLP techniques can be applied for sentiment analysis to gauge patient feedback or emotional states, and for topic modeling to uncover prevalent themes or trends in patient histories. Through these advanced methods, text data can be converted into structured information, which can then be integrated with other types of features to improve the overall risk prediction.

Time-series data, such as time-series data from ECG readings or continuous health monitoring devices, presents unique challenges due to its sequential nature. Raw time-series data can be stored and directly analyzed, allowing deep learning models to work with the data without the need for predefined feature engineering. This direct approach opens up the potential to uncover new patterns and relationships that may not be captured by traditional feature extraction methods. Time-series data is typically processed using models designed to handle sequential data, such as Recurrent Neural Networks (RNNs) and Long Short-Term Memory (LSTM) networks, which excel at capturing temporal dependencies. These models are particularly useful for predicting future events based on past patterns-such as estimating the risk of conditions like atrial fibrillation from ECG data. Furthermore, attention mechanisms integrated into RNNs or self-attention models can enhance the understanding of temporal dependencies, enabling the model to focus on the most relevant time points without being constrained by sequential processing. For more complex signal structures, Spectral Temporal Graph Neural Networks (STGNNs) can be employed, combining graph-based learning with spectral analysis to model intricate relationships within the time-series data. If the volume of user data is large enough, deep learning models can be trained directly on raw time-series data, allowing them to automatically learn to extract relevant features and improve predictive accuracy.

The first model may further include at least one intermediate layer configured to concatenate the features extracted by each input layer. Concatenation can be achieved through several methods.

Flat Concatenation: after the individual features are extracted for each data type, they can be flattened into one-dimensional vectors and concatenated into a single feature vector. For example, features from numerical data (e.g., blood pressure readings) could be concatenated with images features (e.g., image features from a CT scan) and time-series features (e.g., heart rate measurements). The resulting vector represents a comprehensive snapshot of the patient's health across multiple data types.

Layer-wise concatenation: the intermediate representations obtained from each input layer can be concatenated at a deeper layer to allow the model to learn complex interactions between the different data sources.

Attention-based Concatenation: in some cases, an attention mechanism can be employed to give different weights to features of different types based on their importance. For example, in predicting the risk of a heart attack, ECG data and image data might be more relevant than patient history, so the model can "pay more attention" to these data types during concatenation.

After concatenating of the features, a feature selection algorithm like LASSO, Random Forest, or mutual information may be used to select the most relevant features and avoid overfitting.

Methods like Principal Component Analysis (PCA) or t-SNE may be applied to reduce the concatenated feature space, making it easier for the model to learn effectively. These dimensionality reduction techniques can help eliminate noise, reduce computational cost, and provide a clearer visualization of the data in a lower-dimensional space, which aids in feature selection and model performance.

After concatenation, the model may include at least one shared dense layer (fully connected layer) where higher-level, abstract features are learned. These layers allow the model to capture complex interactions across different types of data, integrating information from numerical, image, text, and temporal modalities into a cohesive representation. Regularization techniques like dropout may be applied to prevent overfitting during this stage.

The model may further include at least one output layer configured to predict a risk score. This output layer processes the concatenated features from all input modalities, to predict a risk score for a particular anomaly (e.g., likelihood of a heart attack, stroke, or cancer). This can be a continuous value (risk score) or a classification (e.g., high-risk, medium-risk, low-risk).

In this approach, cross-modal learning techniques like Canonical Correlation Analysis (CCA) or Joint Embeddings may be used to correlate features from different modalities and improve model learning. These techniques allow the model to find common representations across different types of data (e.g., image and time-series data), which can improve the overall prediction accuracy and uncover deeper insights.

To ensure optimal training of the multi-input model, a loss function may be employed to quantifies the difference between the model's predictions and the true values. The loss function helps the model minimize prediction errors and improve performance over time through iterative weight updates via gradient descent or other optimization algorithms.

In the case of the multi-input model, where multiple data modalities contribute to the final prediction, a composite loss function may be employed. Given the heterogeneous nature of the input data, the loss function may consist of multiple terms, each corresponding to a specific sub-task within the model. Notably, the loss function may be designed to account for both feature extraction quality and risk score prediction accuracy. For instance, if the model incorporates separate input layers for numerical, textual, and image data, individual loss components may be defined for each input layer (e.g., MSE for numerical features, cross-entropy loss for categorical text features, and contrastive or triplet loss for embeddings from image data). These individual loss components may be weighted and combined to form a global loss function that guides the optimization process. Additionally, if feature extraction is a separate learning objective from the risk score prediction objective, regularization terms or auxiliary losses may be incorporated to encourage meaningful representation learning. The loss function may comprise a comparison of the risk score prediction and a ground truth risk score using an appropriate metric, such as MSE or cross-entropy. The weights of the different loss components may be adjusted dynamically to balance the learning contributions of each input modality, ensuring that no single data type dominates the training process.

After training, a model validation may be performed using statistical techniques, including cross-validation, receiver operating characteristic (ROC) curve analysis, and confusion matrices, ensuring the reliability and accuracy of the predictive models. New models may be benchmarked against existing published models to verify their performances.

Finally, data storage will be secured in compliance with regulatory standards such as GDPR or HIPAA, utilizing encrypted cloud storage or on-premises data warehouses. A data lake architecture may be implemented to manage large-scale, diverse data efficiently, ensuring accessibility for future analytical advancements.

Device 100 may further comprise a module 150 for providing the trained model for prediction 31.

In its automatic actions, the device 100 may for example execute the following computer-implemented method 400 (illustrated on **Figure 3****)** for obtaining a trained model for prediction 31 of at least one risk score of a user for developing an abnormality with respect to a health status of the user, the method 400 comprising:
- receiving 410 at least one user data 21, measured and/or obtained from said user, and at least one reference data 22, both said user data 21 and said reference data being related to said abnormality;
- generating 430 a training dataset using said at least one user data 21 and said at least one reference data 22;
- obtaining 440 said trained model for prediction 31 by feeding said training dataset to a first model, wherein said trained model for prediction 31 is configured to receive as input at least one current user data 23 and provide as output said at least one risk score of the user for developing said abnormality;
- providing 450 said trained model for prediction 31.

The present disclosure further relates to a device 200 for assisting the user by providing at least one health-related instruction for improving a health status of the user, as illustrated on **Figure 2****.**

Device 200 may be configured to receive at least one current user data 23 and output at least one health-related instruction 32.

The health-related instruction 32 is a directive or recommendation related to the user's health. It may be a proposition to undergo a medical examination, an invitation to schedule an appointment with a healthcare professional, an advice to modify a lifestyle habit, an instruction to trigger an alert, an instruction to control a peripheral device, or an instruction to establish a communication with another user.

The health-related instruction 32 may be formatted as structured data (JSON, XML, key-value pairs), natural language text, encoded numerical or categorical outputs, device control commands, or multimodal outputs (text, audio, visual cues), depending on the application and system requirements.

This health-related instruction 32 may be used directly by the user or by a health professional to guide medical decision-making, adjust treatment plans, schedule necessary interventions, monitor health conditions, trigger alerts for critical health events, control medical devices, or facilitate communication between patients and caregivers for improved healthcare management.

Device 200 may comprise a module 210 configured to receive, from one or more local or remote database(s) 10, at least one current user data 23. The database(s) 10 can take the form of storage resources available from any kind of appropriate storage means, which can be notably a RAM or an EEPROM (Electrically-Erasable Programmable Read-Only Memory) such as a Flash memory, possibly within an SSD (Solid-State Disk). Alternatively, the at least one current user data 23 may be received from a communication network. According to an embodiment, some input(s) may be stored in one or more local or remote database(s) 10 and other (s) may be received from a communication network.

A current user data 23 may be at least one of a data collected by the healthcare system, a data provided directly by the user, and data derived from other external sources. The current user data 23 may comprise at least one information or at least two information.

The current user data 23 may comprise demographic information (e.g., the user's name, age, sex, height, weight etc.), the health history (e.g., past diseases, current medication, vaccination history), clinical examination results (e.g., biological samples analysis results, imaging data, time series data, genomic or omics data), lifestyles factors (e.g., tobacco use, alcohol consumption, physical activity). Furthermore, the data may reflect aspects of the user's mood, energy levels, sleep patterns, diet and mental health (e.g., stress, anxiety).

The current user data 23 may be in various formats (e.g., text, image, audio, video etc.).

The current user data 23 may be collected through structured question-and-answer formats (e.g., Yes/No responses) or through patient-reported information via surveys or apps, using wearable sensors (e.g., personal wearable devices like fitness trackers or smartwatches or medical wearable device such as blood pressure monitors or glucose meters), diagnostic devices (MRI, CT scan, X-ray etc.). Additional sources may include social media data, environmental sensors, or data from Internet of Medical Things (IMoT) devices that track daily activity, sleep, or environmental conditions.

The current user data 23 comprises at least one information that can be linked to an abnormality. For instance, a single information may deviate from typical results, or multiple information may be correlated to identify such deviations. By monitoring and correlating data from multiple sources, deviations from typical physiological behavior can be detected, potentially indicating physical, physiological, symptomatic, diagnostic, behavioral, or pathological abnormalities that may require further investigation or intervention.

The current user data 23 may be preprocessed in the same manner as described with reference to module 120.

Device 200 may comprise a module 220 configured to calculate at least one risk score of the user for developing the abnormality using the trained model for prediction obtained by the device 100.

The risk score may be a probability value that indicates the likelihood of an event occurring within a specified time period (e.g., 20% chance of developing a certain condition in the next 10 years).

The risk score may be a standard deviation from population norms: a risk score presented as a number of standard deviations away from the mean of a reference population. This indicates how much an individual's risk deviates from the average.

The risk score may be a binary value: a simple yes/no or high/low risk classification. This is less nuanced but may be easier for patients to understand.

The risk score may be a categorical value: risk categories such as low, moderate, and high. This method categorizes individuals into discrete risk groups based on their risk score.

The risk score may be a gauge with risk height: a visual gauge or meter that shows the individual's risk level, often color-coded (e.g., green for low risk, yellow for moderate risk, red for high risk).

The risk score may be a comparison of Physiological Age to Chronological Age: an assessment that compares an individual's physiological or 'biological' age (which reflects health status) to their chronological age, indicating if their body is aging faster or slower than average.

The risk score may be a probability of having a disease: an estimate of the current probability that an individual has a disease, often based on diagnostic test results and symptom assessments.

The risk score may be a risk trending over time: a graph or chart showing how an individual's risk has changed over time, which can be useful for tracking the impact of lifestyle changes or treatments.

The risk score may be a threshold-based alert: automated alerts that are triggered when risk scores surpass a certain threshold, indicating a need for clinical attention.

For example, different types of risk scores may be combined to provide a comprehensive risk assessment for the different organs or potential pathology. The choice of format for communicating risk is often guided by the goal of making the information actionable and understandable for both healthcare providers and patients. Visual aids like gauges or charts can be particularly effective for quickly communicating complex risk information in an intuitive way. Spider chart comparing the physiological age to chronological age for different organ or function could be used a summary of the evaluation.

Device 200 may comprise a module 230 for providing as input to a trained second model said at least one risk score to obtain as output the at least one health-related instruction.

The trained second model may be previously trained using a database comprising at least risk scores and associated instructions.

For instance, the training dataset may be constructed using data (data on risks scores and data on instructions and data on potential relationship between both) collected from expert-annotated databases and/or through synthetic data generation. The collected data may be labelled to directly map risks scores and instructions (supervised learning) or by leveraging unlabeled or weakly labeled data for semi-supervised learning or using expert validation feedback (reinforcement learning).

The second model architecture may be a transformed-based model (e.g., BERT, T5), a hybrid NN (configured to take numerical input and output text), a Graph Neural Network, a RNN or a LSTM.

A loss function may be implemented (e.g., cross-entropy loss, contrastive loss, Seq2Seq loss) to optimize training.

Device 200 may further comprise a module 240 for providing the at least one health-related instruction 32.

A command may be associated with the health-related instruction 32.

The device 200 may further comprises at least one graphical interface to display a virtual model of the user.

For example, a virtual model of the user may be an avatar model or a digital twin model obtained using the trained machine learning model 31 and/or the trained second model.

The virtual model of the user may be obtained using a biostatistical model.

The biostatical model may be a model of an individual user that allows comparison to populational data.For example, the virtual model of the user creation may involve constructing a digital replica of the user's health status using insights gained from a thorough longitudinal analysis of the user's data. This virtual model of the user is dynamic and personalized enabling the simulation of various health scenarios, providing a powerful tool for risk assessment and preventive health management. The virtual model of the user thus may serve as a crucial element in a larger system designed to monitor, predict, and guide health prevention strategies tailored to the individual. Integration with Clinical Scores: the virtual model of the user may be initiated by incorporating existing, validated clinical scores (previously obtained risk scores, e.g., obtained using the trained machine learning model 31) pertinent to the user's health conditions;
- Advantagesouly, these scores offer a more precise risk assessment and insight into the progression of diseases, as they are based on clinical research.

### Longitudinal Data Analysis:

- The patient's historical and current health data are examined over time to identify patterns and trends.
- This analysis aids in understanding the progression of the patient's health and in making predictions about future health trajectories.

### Continuous Update and Validation:

The virtual model of the user is not static; it is updated continually with new patient data. The virtual model of the user is regularly revalidated against the latest clinical scores to ensure its accuracy and relevance over time.

### Construction of the virtual model of the user:

The construction of the virtual model of the user involves a combination of approaches based on data from physiology, biology, and pathology, enriched by machine learning and statistical modeling techniques. The virtual model of the user may be created based on initial measurements and longitudinal user follow-up. This representation reflects the user's current state of health and can project potential future states according to various scenarios. The virtual model of the user may predict disease risk (i.e. at least one risk score of the user for developing an abnormality). It enables different types of data to be integrated, providing information on different organs or functions in a single representation.

The virtual model of the user may be developed through a sequence of steps:
i. Initializing with Literature-Based Population Data: The virtual model of the user may be developed starting with a knowledge-based algorithmic approach using existing literature data on risk factors for diseases. These literature data serves as a reliable starting point for estimating individual risk and directing further investigation.
ii. Personalizing with Patient-Specific Data: patient-specific data is integrated. Real-time patient data and historical trends are analyzed to identify individuals at high risk for diseases, allowing for the recommendation of preventive measures.
iii. Continuously Refining the Model with Changes in Risk Factors: The virtual model of the user is designed to refine its predictions and interventions based on continuous input from two distinct systems: a) Modeling based on known data from the literature, and b) Learning from the cohort data collected over time.

This hybrid approach ensures safety limits for data-driven models and provides a control framework to mitigate biases that may arise from non-representative populations.

Throughout this process, the virtual model of the user may employ a combination of statistical and machine learning analyses to refine models as new data on the target population is collected. By adopting this approach, the virtual model of the user m evolves to provide a dynamic, personalized assessment of health risks and adapts treatment to the individual patient's response to intervention.

### Algorithmic Refinement with ML:

- Machine learning techniques (e.g. first model, second model) may be employed to enhance the predictive algorithms. These algorithms are trained on the combined dataset of longitudinal health data and clinical scores.
- Such training enables the algorithms to detect complex patterns in the data, resulting in more accurate predictions.

### Continuous Update and Validation:

- The virtual model of the user is not static; it is updated continually with new user data (for instance, using : the at least one user data, the at least one current user data).
- The virtual model of the user is regularly revalidated against the latest clinical scores (e.g. from new literature data) to ensure its accuracy and relevance over time.

This dynamic, comprehensive, and personalized model enables the simulation of various health scenarios, providing a powerful tool for risk assessment and preventive health management. The virtual model of the user thus serves as a crucial element in a larger system designed to monitor, predict, and guide health prevention strategies tailored to the individual.

The algorithmic foundation to design of a virtual model of the user for health prevention and risk evaluation, based on medical data, would involve several key steps. This application would require tailoring the algorithms to handle health-related data and outcomes. This virtual model of the user would offer a powerful tool for personalized health management, enabling proactive interventions and optimizing health outcomes based on a comprehensive, data-driven understanding of an individual's health status and risks. Here's a detailed breakdown:
Abstraction and Modeling of Health Parameters
   - Data Sources: Integrate data from electronic health records (EHRs) and a dedicated mobile application that will help to collect data directly from patient. This could be extended to wearable health trackers, genetic testing, and environmental sensors.
   - Health Parameters: Define a comprehensive set of health parameters, including physiological metrics (e.g., heart rate, blood pressure), lifestyle factors (e.g., activity level, diet), environmental exposures, and genetic predispositions (see parameters).
   - Probabilistic Graphical Model (PGM): Develop a PGM that encapsulates the complex interactions between these health parameters and their impact on an individual's health status. This model serves as the mathematical foundation of the virtual model of the user, representing the abstracted 'physical state' of an individual.
Bayesian Inference for Data Integration
   - Real-Time Data Assimilation: Employ Bayesian inference to assimilate real-time data from various sources, updating the virtual model's state to reflect the latest health information.
   - Predictive Analytics: Use the virtual model of the user to predict potential health outcomes, identifying risks based on current health data and historical trends.
Dynamic Decision Networks for Health Optimization
   - Decision-Making Framework: Incorporate Dynamic Decision Networks (DDNs) within the PGM to evaluate potential interventions and their impacts on health outcomes. This could involve simulating different scenarios, such as lifestyle changes or medication adjustments, to find optimal prevention strategies.
   - Personalized Health Recommendations: Generate personalized health recommendations based on the DDN's analysis, aiming to minimize risk factors and improve overall health. Calibration and Continuous Updating
   - Model Calibration: Use advanced algorithms, such as sum-product algorithms for efficient inference, to calibrate the virtual model of the user based on initial data and ongoing updates. This may involve machine learning techniques to refine the model's predictions over time.
   - Continuous Monitoring and Updating: Implement a system for continuous data collection and model updating, ensuring the virtual model of the user remains accurate over time. This involves recalibrating the model as new health data becomes available, allowing for dynamic adjustment of prevention and intervention strategies.
Health Monitoring and Intervention
   - Monitoring: Leverage the virtual model of the user for continuous health monitoring, using predictive analytics to identify early signs of potential health issues.
   - Intervention and Prevention: Based on the model's predictions, provide timely interventions aimed at preventing adverse health outcomes. This could include medical interventions, lifestyle adjustments, or environmental changes.

The multi-modal source fusion method for a health prevention virtual model of the user based on the probabilistic graphical model (PGM). The method used to achieve multisource or multimodal integration can be described as follows:

Data collection (as detailed above): Collecting data from multiple sources such as personal and family medical history, biology data, medical test results, wearable devices (MIoT), medical records, genetic information and environmental data.

Feature extraction (as detailed above): Pre-process data to extract relevant features that can be used for modeling. This can include normalization, dimensionality reduction and the treatment of missing or uncertain data.

Model development: Create a PGM that represents the health state and the factors that affect it. This model includes nodes representing the various health parameters and edges representing the probabilistic relationships between them.

Data assimilation: Use Bayesian inference within the PGM to assimilate new data as it becomes available, updating the state of the virtual model of the user. This step should use algorithms for sequential data assimilation.

Prediction: Use PGM to predict future health states and outcomes. This could be achieved by using simulation techniques or inferential statistics to estimate the probabilities of different future states.

Decision-making: Implement dynamic decision networks (DDNs) on top of the PGM to model potential interventions and their impact on predicted health outcomes. This could involve stochastic optimization techniques to find optimal intervention strategies.

Continuous learning and updating: As new data are collected, the first, second and/ or virtual model needs to be continually updated. Machine learning algorithms can be used to refine model parameters based on new data, and the model structure can be revised if necessary to better capture relationships between health parameters.

Quantifying uncertainty: Since health data can be noisy and uncertain, the model(s) must take this uncertainty into account. Robust statistics or Bayesian statistics techniques can be used to quantify and manage uncertainty in predictions and decisions.

Integration and visualization: Finally, the virtual model of the user needs to integrate the model's data and results to create an overall view of the individual's health. Visualization tools can be used to represent health status and predictions in an interpretable way.

These steps would be underpinned by computer algorithms that facilitate efficient PGM calculation, manage large datasets and provide scalable solutions to enable real-time updates and predictions. Specific algorithms such as Monte Carlo methods, Markov chain Monte Carlo, variational inference and deep learning models for pattern recognition in complex datasets could also form part of the multimodal fusion approach.

In a preventive health, the virtual model of the user may be updated through a process that involves continuous data assimilation and model recalibration. The frequency of avatar updates can vary depending on several factors, such as the nature of the data, the specific health conditions being monitored and the objectives of the virtual model of the user.

The process is data-driven and depends on the design of the virtual model of the user, available IT resources and the specific requirements of the individual's health monitoring plan. The aim is to maintain an up-to-date and accurate representation of the individual's health status, enabling timely and informed health management decisions. The following paragraphs provides an example of general process for updating the avatar (i.e., virtual model):
1. Data assimilation: Real-time health data from wearable devices, medical tests or user-supplied information is collected each time a new measurement is taken. This can include physiological measurements such as heart rate, blood pressure, blood sugar, activity data, etc. New blood tests, or information collected directly from the patient about his or her lifestyle.
2. Model recalibration: Incoming data is used to update the probabilistic graphical model (PGM) underlying the avatar. Techniques such as Bayesian updating could be used to assimilate the new information and adjust the model's predictions and state estimates accordingly.
3. Frequency of updates: The frequency of avatar updates can vary according to the type of data on the one hand, and individual risks on the other:
   - High-frequency updates (hourly or daily): For rapidly changing parameters or critical health conditions that require close monitoring, the avatar can be updated almost in real time, potentially every few minutes or seconds. This type of data would mainly come from embedded sensors, such as connected watches, blood pressure monitors or scales, voice analysis, diet information or environmental sensors.
   - Regular updates (weekly or monthly): For routine monitoring, weekly or monthly updates may be sufficient, depending on changes in health parameters and activities, and changes in risk factors.
   - Periodic updates: For long-term health monitoring, updates can be made annually at the time of a new health check-up, for example.
4. Triggered updates: In addition to scheduled or automatic updates, certain events can trigger an immediate update of the avatar. For example, the detection of an anomaly in the data (such as a sudden increase in heart rate) may trigger an urgent reassessment of the person's state of health.
5. Machine learning and adaptation: Advanced virtual model of the user can incorporate machine learning algorithms that can learn from the data over time, leading to a more refined model that can adapt to changes in the individual's health patterns and improve the accuracy of predictions.
6. Feedback loop: The virtual model of the user can include a feedback mechanism whereby the results of interventions (e.g. medication intake, lifestyle changes) are monitored to assess their effectiveness, which then influences subsequent updates to the model and avatar.

Risk scores for various diseases may be integrated into a virtual model of the user for comprehensive risk assessment:
Cardiovascular Disease: Framingham Risk Score, QRISK, SCORE and SCORE 2, Reynolds Risk Score, etc.
Diabetes: A1C Test, Diabetes Risk Score (Finnish Diabetes Risk Score - FINDRISC) Stroke: CHADS2 Score, NIH Stroke Scale
Osteoporosis and Related Fracture Risk: FRAX Score, Bone Mineral Density Testing Fall Risk in the Elderly: Morse Fall Scale, Timed Up and Go Test (TUG)
Memory Disorders and Dementia: Mini-Mental State Examination (MMSE), Montreal Cognitive Assessment (MoCA)
Stress-Related Depression: Patient Health Questionnaire-9 (PHQ-9), Beck Depression Inventory (BDI)
Cancers: Gail Model (for breast cancer), Prostate Cancer Risk Calculator, PSA density, etc.
Respiratory: BODE Index for Chronic Obstructive Pulmonary Disease (COPD), Asthma Control, Test (ACT)
Renal:Chronic Kidney Disease Epidemiology Collaboration (CKD-EPI) Equation Nutrition and Metabolism: Body Mass Index (BMI), Waist-to-Hip Ratio (WHR) Geriatrics: Activities of Daily Living (ADL) Scale, Geriatric Depression Scale (GDS) These risk scores may be based on large-scale studies and can be adapted or integrated into a virtual model platform to provide personalized risk assessments for patients.
The risk scores listed may be obtained from a combination of clinical data, patient-reported outcomes, and sometimes genetic information. They may be designed to estimate the risk of developing a specific condition or the likelihood of an adverse event occurring. Here's is described an exemplary overview of how these scores may be obtained and the types of parameters they integrate:
1. Data Collection: Risk scores may be typically derived from patient data that may include demographic information (age, sex), clinical measurements (blood pressure, cholesterol levels), health behaviors (smoking status, physical activity), and medical history (previous diseases, family history).
2. Statistical Analysis: This data may be analyzed using statistical models that have been developed and validated through large-scale studies or clinical trials. These models identify patterns and correlations between various factors and the outcome of interest (e.g., heart attack, stroke).
3. Algorithm Application: The final risk score may be calculated by inputting an individual's specific data into an algorithm that has been derived from these models. The algorithm assigns weight to different factors based on how strongly they predict the outcome.
4. Risk Estimation: The output may be a score that represents the estimated risk of the individual developing the condition/abnormality or experiencing the event within a certain time frame (e.g., 10 years for cardiovascular risk).

As an example, the SCORE2 risk assessment for cardiovascular diseases may be detailed as follows:

The SCORE2 risk prediction algorithm is based on statistical models that estimate sex-specific associations, known as subdistribution hazard ratios (SHRs), which are adjusted for competing risks and stratified by cohort. The variables used in the SCORE2 models include: age, current smoking status, history of diabetes mellitus, systolic blood pressure, total cholesterol and HDL cholesterol. These factors were chosen for their strong predictive ability and availability in derivation cohorts, target populations for screening, and population statistics needed for model recalibration. The models account for age interactions because the association of these risk factors with cardiovascular disease (CVD) declines with increasing age. The SCORE2 models are intended for use in individuals aged 40-69 years without previous CVD. The risk models were recalibrated to risk regions using age- and sex-specific mean risk factor levels and CVD incidence rates. European countries were grouped into four risk regions based on their most recently reported WHO age- and sex-standardized overall CVD mortality rates:
- Low risk: <100 CVD deaths per 100,000 population
- Moderate risk: 100 to <150 CVD deaths per 100,000 population
- High risk: 150 to <300 CVD deaths per 100,000 population
- Very high risk: ≥300 CVD deaths per 100,000 population

Incidence rates for these regions were estimated by rescaling region-specific CVD mortality rates with derived age, sex, and region-specific multipliers from contemporary representative cohorts.

By integrating multiple risk scores into a virtual model of the user, healthcare providers can achieve a nuanced understanding of a patient's health, leading to better-informed clinical decisions and improved patient outcomes. This holistic approach also allows for the anticipation of health risks and the proactive management of potential issues.

### Score Calculation:

- Calculate individual risk scores (like SCORE2 for cardiovascular risk, FINDRISC for diabetes, FRAX for osteoporosis, etc.) using the specific algorithms for each condition.
- Ensure that the data used for each score is current and accurately reflects the patient's present health status.

### Aggregation and Weighting:

- Aggregate the results of these individual scores. This could involve creating a weighted index if certain conditions are of more concern due to the patient's history or genetic predisposition.
- Use expert systems or clinical guidelines to determine the relevance and weight of each score.

### Probabilistic Graphical Model (PGM):

- Develop a PGM that includes nodes for each of the calculated risk scores and their associated health outcomes.
- Model the interactions between these risk scores to understand the compounded or mitigating effects of one condition on another.

### Dynamic Health Profile:

- Update the virtual model of the user dynamically with new data to reflect changes in the risk scores over time.
- Model the effects of lifestyle changes, interventions, or treatments on these scores to provide a forecast of health outcomes.

### Risk Stratification and Personalization:

- Use the integrated risk scores to stratify the patient into different risk categories for various health outcomes.
- Personalize health recommendations based on the stratification and the patient's unique health profile.

### Decision Support:

- Implement a decision support system within the virtual model of the user that can interpret the aggregated risk scores and provide recommendations for prevention, monitoring, or treatment strategies.

### Visualization and Reporting:

- Use visualization tools to present a unified view of the patient's health risks to healthcare providers, enabling easy interpretation and decision-making.
- Provide reports or dashboards that highlight key risks and potential areas for intervention.

### Continuous Learning:

- Apply machine learning algorithms to analyze the outcomes of previous interventions and refine the virtual model of the user model for better prediction and personalization.
- Use the feedback from these outcomes to adjust the weight and impact of individual risk scores within the virtual model of the user.

### Patient Engagement:

- Engage patients by providing them with access to their health profiles and risk scores, empowering them with knowledge and involving them in their health management.

In constructing the virtual model of the user, a multifaceted algorithmic approach may be employed, encompassing a spectrum of methodologies tailored to the nature of the data. For continuous and categorical data, foundational biostatistical methods such as linear and logistic regression may be used to understand and quantify relationships. For structured data analysis, machine learning techniques like Support Vector Machines (SVMs) and ensemble methods including Random Forests and Boosted Decision Trees (e.g., XGBoost, LightGBM) may be utilized to handle complex and high-dimensional datasets.

The avatar within the virtual model of the user may be developed using Probabilistic Graphical Models (PGMs) to encode the conditional dependencies between various health factors. Bayesian Inference may be used allowing for the integration of new data and the updating of the avatar's state in a principled manner. Additionally, Dynamic Decision Networks (DDNs) may be integrated to provide the system with the capability to make informed decisions and predict future health trajectories based on current (e.g. current user data) and past data (e.g. user data).

Deep Neural Networks may be applied where the data exhibits intricate structures or nonlinear relationships that simpler models cannot capture effectively. This includes Convolutional Neural Networks (CNNs and 3D CNNs) for the analysis of medical imaging data and Recurrent Neural Networks (RNNs) for time-series data like ECG or continuous health monitoring data. The choice between applying machine learning to extracted features or directly to raw data may depend on the specific characteristics and quality of the data source.

The fusion of these algorithms may enable the virtual model of the user to provide a dynamic and detailed representation of the patient's health, integrating diverse data streams into a coherent model that can evolve and adapt over time.

Examples of algorithms that may be used:
1. For Biostatistical Analysis:
   - Generalized Linear Models (GLMs): Extend linear and logistic regression to model various types of data distributions.
   - Cox Proportional Hazards Model: For survival data, this model can estimate the time until an event of interest, like the onset of a disease.
   - Bayesian Hierarchical Models: They allow for the incorporation of different levels of information and can handle variability between patients and groups.
2. For Structured Advance Data Analysis:
   - Decision Trees: A simple alternative to Random Forests, offering more interpretability at the cost of some predictive power.
   - Nearest Neighbors: A non-parametric method that classifies based on the closest training examples in the feature space.
3. For Deep Neural Networks:
   - Autoencoders: For unsupervised learning tasks, they can help in feature extraction and dimensionality reduction.
   - Transformer Models**: Especially useful for sequential data,
4. For Probabilistic Graphical Models:
   - Markov Random Fields: They are an alternative to Bayesian Networks, suitable when the relationships between variables are undirected.
5. For Dynamic Decision Networks:
   - Markov Decision Processes (MDPs) or Reinforcement Learning (RL):
6. For Time-Series Analysis:
   - **ARIMA Models (Autoregressive Integrated Moving Average)**: Traditional statistical models for forecasting.
State Space Models**: Can capture complex time-varying patterns in time series data. The choice of algorithm may depend on specific use-case requirements, including the interpretability of the model, the type of data, the computational efficiency, and the predictive performance. It's also common to use a combination of these algorithms in a single virtual model of the user to leverage their strengths. For instance, a virtual model of the user may use a GLM for initial risk stratification, a deep learning model for image analysis, and a PGM for overall health state representation and decision-making.

For example, the medical/historical data may provide the foundational inputs for calculating risk scores, which in turn serve to update and refine the virtual model of the user , thereby creating an evolving model of the patient's health that can inform clinical decision-making and patient behavior. Here's a detailed explanation of the process:
1. Data Collection (see above)
2. Risk Score Calculation (see above)
3. Avatar Update:
   - The calculated risk scores are used to update the patient's avatar in the virtual model of the user.
   - The avatar is a virtual representation of the patient's health status, which reflects the current risk assessments for various health conditions.
   - The update process involves integrating the new risk scores into the avatar's profile, which may change the visual representation, or the health indicators associated with the avatar.
4. Dynamic Interaction:
   - The virtual model of the user is dynamic, meaning it can simulate how changes in behavior, treatment, or other interventions might affect the patient's risk scores and, consequently, their health status.
   - For example, if the patient stops smoking, this change can be input into the virtual model of the user, which will then adjust the cardiovascular risk score accordingly.
5. Feedback Loop:
   - The virtual model of the user provides a feedback loop for both the patient and healthcare providers.
   - As interventions are applied and new data is collected (such as follow-up medical tests), the avatar is updated to reflect these changes.
   - This allows for a real-time assessment of how effective interventions are and can guide further treatment decisions.
6. Continuous Monitoring and Updating:
   - The virtual model of the user is designed for continuous monitoring and updating.
   - As new data comes in, risk scores are recalculated, and the avatar is updated, providing an up-to-date holistic view of the patient's health.
7. Decision Support: The virtual model of the user can also act as a decision support tool, using predictive modeling to forecast potential future health scenarios based on current data and trends. This can help in planning preventative measures or preparing for potential health issues before they become serious.

In one or several embodiments, within the device 200, the health-related instruction is displayed to the user on the graphical interface, and the command associated with the health-related instruction consists in at least one of the following:
- sending a notification to the user related to a health status of the user (e.g., blood pressure alert, medication reminder, physical activity reminder, hydration reminder, sleep quality tip, heart health tip, stress management tip, blood glucose alert, weight management tip, health screening reminder) ; and/or
- displaying a report to the user related to a health status of the user (e.g. graph, chart, text document, figure, illustration, map); and/or
- sending a control signal to at least one peripheral device (e.g., digital signal, analog signal, electromagnetic signal); and/or
- sending a control signal to at least one receiving interface such as a mobile application, a telemedicine platform or an online platform.

In its automatic actions (illustrated on **Figure** 4), the device 200 may for example execute the following computer-implemented method 500 assisting the user by providing at least one health-related instruction 32 for improving a health status of the user, said method 500 for assisting comprising:
- receiving 510 at least one current user data 23;
- calculating 520 at least one risk score of the user for developing said abnormality using a trained model for prediction 31 obtained by the device 100 according to any of the herein disclosed embodiments;
- providing 530 as input to a trained second model said at least one risk score to obtain as output said at least one health-related instruction, said trained second model being previously trained using a database comprising at least risk scores and associated instructions;
- providing 540 said at least one health-related instruction 32.

A particular apparatus 9, visible on **Figure 5****,** is embodying devices 100 et 200 described above. It corresponds for example to a workstation, a laptop, a tablet, a smartphone, or a head-mounted display (HMD).

That apparatus 9 comprises the following elements, connected to each other by a bus 95 of addresses and data that also transports a clock signal:
- a microprocessor 91 (or CPU);
- a graphics card 92 comprising several Graphical Processing Units (or GPUs) 920 and a Graphical Random Access Memory (GRAM) 921; the GPUs are quite suited to image processing, due to their highly parallel structure;
- a non-volatile memory of ROM type 96;
- a RAM 97;
- one or several I/O (Input/Output) devices 94 such as for example a keyboard, a mouse, a trackball, a webcam; other modes for introduction of commands such as for example vocal recognition are also possible;
- a power source 98; and
- a radiofrequency unit 99.

According to a variant, the power source 98 is external to the apparatus 9.

The apparatus 9 also comprises a display device 93 of display screen type directly connected to the graphics card 92, via a bus 930, to display synthesized images calculated and composed in the graphics card. The use of a dedicated bus to connect the display device 93 to the graphics card 92 offers the advantage of having much greater data transmission bitrates and thus reducing the latency time for the display of images composed by the graphics card,. According to a variant, a display device is external to apparatus 9 and is connected thereto by a cable or wirelessly for transmitting the display signals. The apparatus 9, for example through the graphics card 92, comprises an interface for transmission or connection adapted to transmit a display signal to an external display means such as for example an LCD or plasma screen or a video-projector. In this respect, the RF unit 99 can be used for wireless transmissions.

It is noted that the word "register" used hereinafter in the description of memories 97 and 921 can designate in each of the memories mentioned, a memory zone of low capacity (some binary data) as well as a memory zone of large capacity (enabling a whole program to be stored or all or part of the data representative of data calculated or to be displayed). Also, the registers represented for the RAM 97 and the GRAM 921 can be arranged and constituted in any manner, and each of them does not necessarily correspond to adjacent memory locations and can be distributed otherwise (which covers notably the situation in which one register includes several smaller registers).

When switched-on, the microprocessor 91 loads and executes the instructions of the program contained in the RAM 97.

As will be understood by a skilled person, the presence of the graphics card 92 is not mandatory, and can be replaced with entire CPU processing and/or simpler visualization implementations.

Device 100 and/or device 200 may be implemented differently than a standalone software, and an apparatus or set of apparatus comprising only parts of the apparatus 9 may be exploited through an API call or via a cloud interface.

### EXAMPLES

The present invention is further illustrated by the following examples.

Example 1: (see **Figure 10****)** Digital platform for predictive and preventive health modeling based on the calculation of individual risks and allowing for the simulation of the impact of treatments or changes in risk factors.

The invention describes an innovative digital health platform that integrates individual virtual twin modeling, artificial intelligence (AI), and predictive analytics to enhance individual health prevention and management. The platform generates digital avatars using personal medical data, lifestyle habits, and physiological measurements to monitor and predict health risks, enabling tailored interventions such as lifestyle recommendations or specific preventive strategies. It also provides interactive visualizations of health risks, compares physiological to actual age, and educates patients in simple, understandable language. This facilitates better communication between patients and caregivers, aiming to shift from a reactive to a proactive and predictive approach to personal health management.

The invention consists of a digital platform for health prevention characterized by the following technical aspects:
1. Modeling system based on the Virtual Twins principle: uses historical and current medical data to build a digital avatar of the patient, providing a dynamic, personalized representation of health status. Real-time dynamic adaptation based on new information obtained from: i) lifestyle changes; ii) new medical test results; iii) measurement sensors (MIoT).
2. Artificial Intelligence and Predictive Analytics: Implements advanced AI algorithms to analyze health data and predict future risks, enabling proactive disease prevention. The results of these algorithms are framed by the integration of existing, validated clinical scores.
3. Data Integration: Collect and combine multi-source data, including physiological measurements, lifestyle habits and environmental parameters, for a comprehensive risk assessment.
4. Risk visualization: Presents an interactive radar graph that compares physiological age with the patient's actual age for different risk factors, making the information immediately understandable.
5. Personalized Recommendations: Offers advice and interventions based on data analysis, such as tailored exercise programs, nutritional advice, and stress reduction strategies.
6. User Interface: Provides an intuitive interface that makes it easy for patients to understand their health and actively participate in their prevention journey.
7. Communication and Education: Facilitates dialogue between patients and healthcare professionals, enabling ongoing monitoring and health education.
8. Interoperability and Scalability: Designed to interface with other healthcare systems and medical devices, ensuring seamless integration and evolution with technological advances.
This combination of features aims to transform individual health management into a more predictive, personalized and preventive process, moving away from traditional reactive and generalist methods of medicine.

Potential inventive processes:
*Innovative modeling method,* detailing the creation of a digital health modeling tool. This tool is noted for its unique integration of the patient's complete personal data, including medical history and test results, to guide preventive healthcare measures.
*The innovative aspect of using virtual twins* lies in their specific application to replicate not just the patient's current health status but also to simulate various preventive scenarios and their potential outcomes.
*The integration of AI for predictive analysis* in prevention and risk assessment involves the development of an advanced artificial intelligence algorithm that can analyze and predict health risks based on collected data. This algorithm represents a technically non-obvious step for an expert in the field.
*Integrate a visual interpretation of risks assessment* using an interactive radar chart that contrasts the physiological age with the actual age of the patient for various diseases and risk factors. This innovative presentation is advantageous because it allows complex medical information to be communicated in a simple and easily understandable way for both patients and doctors.
*Digital prevention platform:* The overall concept of a digital platform that not only monitors, but also predicts and guides health prevention through personalized recommendations based on real-time data.
*Method of communicating with patients:* The ease of use and ability of the tool to educate and inform patients about their health in a way not previously available.

The methodology for creating digital models (virtual twin) for personal health, which appears to be a novel approach in this sector. The application of AI for individualized predictive risk analysis based on a variety of health data, and its ability to adapt to temporal changes. Advanced AI algorithms are implemented to analyze health data and predict future risks. These algorithms are enhanced by the integration of existing validated clinical scores. The creation of a virtual twin based on longitudinal data analysis involves the following steps:
*Data Collection:* a comprehensive questionnaire is administered to collect exhaustive information about personal and family medical history, lifestyle factors including nutrition, tobacco and alcohol use, exercise, stress and anxiety, and sleep patterns. Clinical exams are conducted to measure weight, height, abdominal circumference, pulse, blood pressure, ECG, and mobility. Blood tests are performed to analyze blood count, lipid profile, diabetes markers, and liver and kidney functions.
*Longitudinal Analysis:* The longitudinal data are then analyzed to detect patterns and trends over time. This analysis helps in understanding the progression of a patient's health and predicting future health trajectories.
*Virtual Twin Creation:* Using the insights gained from the longitudinal analysis, a virtual twin or a digital replica of the patient's health is created. This model represents the patient's current health status and can simulate potential future states based on different scenarios.
*Integration with Clinical Scores:* The virtual twin is enhanced by incorporating existing validated clinical scores that are relevant to the patient's health conditions. These scores are based on clinical research and are used to provide a more accurate assessment of risk factors and disease progression.
*Algorithm Enhancement:* The predictive algorithms are refined using machine learning techniques, which are trained on the aggregated longitudinal and clinical score data. This training allows the algorithms to recognize complex patterns and make more precise predictions. The collected data is used to calculate the risk of various health conditions such as cardiovascular diseases (including stroke), diabetes, cancer, depression, memory disorders, sleep issues, mobility and fall risk, and sensory impairments like hearing and vision loss, etc.
*Continuous Update and Validation:* The virtual twin is continuously updated with new patient data and revalidated against updated clinical scores, ensuring the model remains accurate over time.

The risk visualization system or "report card" which would include a risk visualization system using radar graphs to compare physiological and actual age in various health scenarios, can be described as follows:
A risk mapping system is created, which involves the development of a radar graph that maps the calculated risks against both the chronological and physiological age of the patient.
The radar graph provides a visual representation of the patient's risk profile across different health domains, making it easier to identify areas of concern and prioritize interventions.
The report card designed through this process provides an at-a-glance view of the patient's health risks, enabling both the patient and healthcare providers to make informed decisions about preventive and therapeutic strategies. The radar graph's visual comparison of physiological versus chronological age offers a unique perspective on the patient's overall health, potentially motivating them to engage in healthier lifestyle choices or adhere to treatment plans. Developing such a visualization system requires multidisciplinary expertise in healthcare, data analysis, and user interface design. It must be intuitive for users while providing actionable insights. The goal is to ensure the system is not only accurate in its assessments but also effective in communicating risk to patients in a way that promotes engagement with their health.

The platform architecture for real-time data integration and longitudinal tracking of patient health. The platform architecture for such a prevention system based on a mobile app, interoperability with existing medical devices or IoT, and cloud-based solutions could be structured as follows:

### Mobile Application (low tech but high ergonomics):

- Serves as the front-end interface for patients and healthcare providers.
- Provides data input capabilities, personalized health dashboards, and risk visualization using radar graphs
- Delivers notifications, reminders, and personalized health recommendations.

### Interoperability:

- Establishes communication with various health devices and wearables through standard protocols like specialized APIs for third-party integration and extension of platform capabilities.
- Integrates with Electronic Health Records (EHR) systems for seamless data exchange.
- Collects data from MioT devices such as blood pressure monitors, fitness trackers, etc.

### Data Processing and Analysis (see above)

### Cloud Infrastructure:

- Supports storage and management of large volumes of health data securely.
- Facilitates scalable computational resources for complex data analysis and machine learning tasks.
- Ensures high availability and redundancy for constant access to the platform.

### Database Layer:

- Stores patient data, medical records, and analysis results in a structured format.
- Uses encryption and other security measures to protect sensitive information.
- Allows for querying and retrieving data for various platform functions.

### Security and Compliance:

- Implements robust security protocols to ensure data privacy and compliance with healthcare regulations like HIPAA or GDPR.
- Manages user authentication, authorization, and audit trails.

### Analytics and Reporting:

- Generates comprehensive reports and insights for patients and healthcare providers.
- Visualizes data through interactive radar graphs and other visualization tools.

### Education Layer:

- Supports messaging, and other communication tools for patient-provider interaction.
- Facilitates remote monitoring and capture of life habitus.
The cross-disciplinary integration of all a patient's medical data for prevention. A comprehensive risk assessment is achieved by collecting and combining multi-source data, including physiological measurements, lifestyle habits, and environmental parameters.

### Holistic Patient Profile:

By combining data from multiple sources, including physiological measurements, lifestyle habits, and environmental factors, a more complete and accurate picture of the patient's health status is formed. This allows for the identification of risk factors that may not be apparent when considering single data sources in isolation.

### Early Detection and Prevention:

Integrating diverse data sources can help detect early signs of potential health issues, allowing for timely preventive measures. For instance, subtle changes in daily activity patterns tracked by wearables may signal emerging health problems that require intervention.

### Personalized Healthcare:

Cross-disciplinary data integration enables healthcare providers to tailor prevention and treatment plans to the individual, taking into account their unique medical background, lifestyle, and environment.

### Example 2:

The goal is to create a personal digital platform dedicated to prevention and personal health monitoring. Based on a unique application that allows individuals and their caregivers to create a digital twin of their risk factors and health status at a particular moment and monitor how they evolve. The avatar is provided with health data, lifestyle information (physical activity, diet, sleep, stress, etc.) and physiological parameters (heart rate, blood pressure, etc.). The platform will analyze this data to provide personalized prevention recommendations, such as screening or targeted interventions to reduce risk factors (setting up an exercise program, nutritional advice, addiction cessation, coaching, improving sleep quality, limiting stress, etc.). The ability to inform patients about their own health and the possibility of changing its course in a simple, transparent and didactic way will be a key component of this tool. As a communication tool, it should enable patients and caregivers to collect health data as they go. It should also facilitate the exchange of information.

Collection of individual data through the creation of an electronic medical record (EMR) for prevention (pEMR). The goal is to provide a digital medium that allows all stakeholders (patients, medical staff, caregivers) to collect and share health data useful for the prevention program. It will be fed from a variety of sources, with a preference for mobile applications. The platform must be able to integrate health data traditionally collected by the health care system (health history, clinical exam, biological, imaging, time series), innovative health data (video, audio, ...), collect measurements from Internet Medical of Things (IMoT), interact with patients to obtain direct information on their lifestyle, and in the near future, genomics data or other omics.

### Ergonomics, mobility, interoperability, simplicity, transparency, security and didactic nature of the information provided will be the challenges in designing this PEMR.

Data collection is longitudinal and incremental (see **Figure** 6). In the first phase, before the patient arrives for the on-site examination, a comprehensive health questionnaire (120 questions) is sent to the patient in a digital format compatible with a mobile application. To enable risk assessment, biological data (approximately 50 parameters) and basic clinical examination data (6-10 parameters) are also needed. The pEMR must be able to: i) accumulate identical data in the form of time series, ii) allow the creation of new fields as science and the project progress.

Modeling and predicting individual risk by analyzing real-time patient data along with historical trends, digital twins can identify individuals at high risk for disease due to high risk factors and recommend secondary (screening) and primary (risk reduction) preventive measures.

Integration and analysis will initially focus on a relatively limited set of data. Risk prediction will involve two distinct systems: i) modeling based on known data from the literature, and ii) learning from our own cohort data.

Numerous epidemiological studies have already scientifically established the risk factors for many diseases. These data are readily integrated and, in some cases, are already available in the form of risk scores for many diseases, including cardiovascular disease, diabetes, stroke, osteoporosis and related fracture risk, fall risk in the elderly, memory disorders and dementia, stress-related depression, cancers, etc.

Not to use these risk factors, which already exist and have often been validated on a very large scale, would be scientifically questionable. Above all, they allow us to "hit the ground running" without waiting to collect our own data. Finally, this scientific work provides a solid framework within which we can validate the results of our learning process.

To promote patient empowerment and education, the twin must help patients understand their health and become active participants in their own care. By giving them full, transparent, and educational access to their health data - including personalized health information, treatment plans, and progress monitoring - citizens can become more engaged in managing their own health. This engagement will lead to better adherence to treatment, essential lifestyle changes, and better self-management of their health. This engagement needs to be reflected in improved health data collection so that models can be updated (monitoring). By facilitating communication and collaboration between patients and caregivers, the tool should also promote shared decision making and empowerment of all stakeholders.

We need to find a simple, visual way to show patients their strengths and weaknesses when explaining risk factors. "Radar" graphs showing their physiological age - for different pathologies or groups of pathologies - as a mirror image of their actual age could be one way to present the results of our models. This presentation of the information also makes it possible to follow the changes over time as a result of the complementary examinations, the drug interventions and the changes in lifestyle. The goal is to provide a holistic view of the health of the client and to convey this information to the client.

**Figure 7** is a presentation of health risks in the form of a radar graph showing the physiological age for a given physiological function (or organ, or pathology) compared to actual age. Temporal evolution of this analysis depending on the tests to be performed and therapeutic measures taken.

The platform needs seamless integration with real-time data from IMoT devices, wearables, and other medical sensors. Continuous monitoring of key metrics and targets will allow deviations from planned goals to be identified and the patient or medical team to be alerted. This will allow health care providers to quickly take charge of the patient and make changes to the treatment or prevention plan. The platform must also be a tool for research and innovation in the field of prevention.

### Example 3: Physical and IT workflow for the checkup and the different software to be design and connected

Each of these stages needs to be carefully designed to ensure user-friendliness, data security, and compliance with health regulations. The technology solution should integrate smoothly with existing hospital information systems and support a range of devices and communication methods.

STEP 1: Appointment process, registration, pre-admission form The ability for patients to manage their appointments and personal health information conveniently and securely will be critical to the success of the application at this step. Here is a step-by-step description of each stage in English, highlighting the key points that would be essential for software development teams to consider when building the application.
1. Email Reception:
   - The patient receives an email containing the appointment date.
   - This email also provides a link to the patient portal web app, where they can manage their appointment and personal medical information.
2. Patient Portal Login:
   - The patient accesses the Hospital patient portal, to manage their login (SSO) link with appointment details.
   - At this stage, the portal should offer a seamless login experience with options for password recovery if needed.
3. Registration Process:
   - The patient registers on the portal, providing personal details such as gender, first and last names, and contact information including telephone or email.
   - This step must ensure data privacy and security, adhering to regulations such as GDPR.
4. Pre-admission Form:
   - The patient fills out a pre-admission form and possibly other specific forms required by the hospital.
   - These forms are to be submitted along with a digital signature, ensuring legal compliance and consent.
   - The first Questionnaire (Q1) should be ideally filled by the patient at this stage
5. Appointment Confirmation:
   - The patient receives a mail validation of the form submission and confirms the detailed program of the day.
   - There should be an option to add the appointment to a digital calendar and upload ID for identification purposes.
6. Appointment Reminder:
   - The day before the appointment, the patient receives an SMS reminder.
   - The system should provide flexibility for the patient to cancel the appointment through the app, SMS, or a phone call.

### STEP 2: Health check journey at hospital

Data flow is a crucial aspect, with patient-provided information, data entry by medical staff, and direct interoperability (interop) of medical results (such as from biology, electrophysiology, radiology, functional exploration, etc.) all needing to be seamlessly integrated.

Here's a step-by-step description of this process, along with the key data points collected at each stage:
1. Arrival at the Check-Up Center:
   - The patient arrives and goes through an identity verification step, which may include the printing of labels for identification purposes and a billing sheet.
   - This stage must integrate with the hospital's administrative system to confirm patient details and facilitate smooth billing processes
2. Pre-admission Questionnaires: (if not done previously at step 1)
   - At the reception, the patient may be required to fill out pre-admission questionnaires on a tablet, which likely includes medical history, current symptoms, and other relevant health information.
   - The application should provide an intuitive interface for entering this information, ensuring it is securely saved and accessible to healthcare professionals.
3. Changing into Medical Attire:
   - The patient changes into the provided attire, usually a gown and T-shirt, to prepare for the medical check-up procedures.
4. Medical Measurements:
   - Initial measurements are taken, such as weight, height, and bioimpedance analysis (body composition).
   - The equipment used here should be able to interface with the digital systems to automatically upload the measurements.
5. Checkup package verification:
   - The healthcare professional (possibly a nurse or technician) verifies the checkup specifics from the patient's medical records, which may be pre-filled based on predetermined checkup packages (type 1, 2, or 3).
   - The software must allow for customization of the checkup based on individual patient needs or corporate health plans.
6. Blood Tests and ECG:
   - The patient undergoes a blood draw and an electrocardiogram (ECG). A lab form is ticked off for the required biological assessments.
   - The integration of these results with the patient's digital health records is crucial, and the system should have functionality to directly import data from lab equipment and ECG machines.
7. Handover of Hemoccult Test:
   - The patient receives a Hemoccult test, which is a screening tool for colorectal cancer.
   - The process should ensure that the patient understands how to use the test, with instructions likely provided digitally for reference.
   - automatic notification of the results to the patient to be integrated.

### STEP 3: Various medical examinations and data collection

The software must handle the diverse data types collected during these examinations and maintain a high level of data privacy and security compliance (see Interop excel file). This includes interoperability with various medical devices and laboratory systems to automate data capture and integration into the patient's digital health records. Here's an outline of the process:
1. Personal Lounge Space:
   - After initial tests, the patient may have a personal space or lounge where they can have breakfast and relax.
   - This area should allow patients to access their health portal to review and select health questionnaires or access other health-related information.
2. Vision and Hearing Tests:
   - The patient undergoes specialized tests such as a vision test and an audiogram.
   - Some of the results from these tests need to be entered by the healthcare professional (possibly a nurse) directly into the tablet, which should be designed for easy data capture.
3. Additional Forms and Questionnaires:
   - Patients are asked to fill progressively when waiting between test various forms related to nutrition, rheumatology/osteoporosis, sleep, psychological well-being (stress, depression, anxiety), cardiovascular health, and gynecological history.
   - These forms should be available digitally, possibly on the patient app, and integrate smoothly with the DPI (Digital Patient Information) system for checkup consolidation. Each patient will receive a Tablet for his checkup journey to complete these forms, and received information
4. Biological Sample Collection:
   - The patient provides samples for various tests such as urine analysis
   - The collection process should be facilitated by a nurse and tracked digitally, ensuring accurate labeling and data entry into the patient's application
5. Imaging Tests:
   - The patient may undergo various imaging tests, such as abdominal-pelvic ultrasound, thyroid ultrasound, mammography, calcium scoring, and CT scans of the lungs.
   - The imaging department (radiology) should have systems in place to directly upload images and reports to the patient's DPI for review and follow-up.
   - Report and images should be automatically imported (Dicom).

### STEP 4: Clinical examinations and report generation

Here's what the process entails, including the examination and consultation phases, and the integration of data into the patient's health records:
1. Waiting Area and Consultations:
   - The patient waits in the designated waiting area before being called in for various consultations.
   - Consultations include cardiology where an interrogation, clinical exam, and tests like an ECG, stress test (Treadmill Test - ETT), and echocardiogram (ETSA) are conducted.
2. Cardiology Data Interpretation:
   - The results from the cardiology tests are interpreted, and relevant health metrics (such as blood glucose, HbA1c, BNP, uric acid, etc.) are recorded.
   - The cardiology report along with recommendations are compiled and integrated into the patient's Digital Patient Information (DPI) system.
3. Gynecological Consultation:
   - A gynecological interrogation and clinical exam take place.
   - Findings are documented and, where applicable, recommendations for vaccinations are made.
4. General Medicine Consultation:
   - The patient undergoes a general medical interrogation and a complete clinical examination.
   - Results from general health metrics (e.g., NFS, biochemistry, immunology, urinalysis) are recorded, and vaccination recommendations are given if necessary.
5. Report Generation and Recommendations:
   - Based on the consultations and examinations, comprehensive reports are generated, and health recommendations are provided.
   - A report template is pre-populated with the results of the different questionnaires, test, and clinical and measurement
   - These reports are fed into the DPI Checkup system and are made available to the patient.
   - A synthetic radar graph of the health check will be automatically provided by the analytics module
   - If necessary additional clinics with more specialized physician could be book. Additional test such as CT coronarography, colonoscopy, etc. could be book in live.
6. Post-Consultation and Dressing:
   - After the consultations, the patient may return to the changing area to get dressed.
   - This step includes personal care such as possible vaccinations and sleep analysis managed by the nursing staff.
7. Report Delivery and Mobile App Integration:
   - The patient receives a printed and physician-signed copy of the consultation report or can access it through the mobile application.
   - This implies the need for the health center's digital system to support printing capabilities and integration with a mobile app platform for patient access.

### Health Check Short Report

This template provides a comprehensive overview of the health check-up, outlining the key components of the summary report and ensuring that all significant health aspects are covered and clearly communicated to the patient.
1. Introduction
   - Date of the check-up
   - Listing of the exams performed
2. Medical and Surgical History
   - Details of past medical and surgical history
3. Risk Factors
   - Listing of identified risk factors
4. Medical Synthesis
   - Implementation of the general practitioner's conclusion
5. Recommendations
   - Suggested exams or consultations based on the check-up results
6. Appointment Schedule
   - List of upcoming appointments at the Hospital with details (Date, Time, Building, Room, Doctor)
7. Communication of Results
   - Recommendation to share all medical check-up results with usual physicians and any doctor consulted subsequently
8. Doctor's Note
   - Availability for further information
   - Electronic signature of the doctor
   - Expressions of best regards
9. Summary
   Global conclusion/synthesis
   Results of the main existing risk scoring system (Cardio, Neuro, cognition, ...)
   AI Global multifactorial risk calculation
   Spider Graph visualization of the risk assessment evaluation
   Vaccination status and recommendations
   Reports from the General Practitioner, Cardiologist (including tests such as ECG, stress test, echocardiogram, TSA echo, calcium scoring, Framingham score), and Gynecologist (including last PAP test, mammography, breast ultrasound)
   Personalized recommendations based on fragility markers
10. Annexes
   Detailed results from biological exams
   Radiological exams (abdominopelvic ultrasound, CT scans, bone densitometry)
   Body composition analysis (impedance)
   Hearing test results (audiogram)
   Vision tests (tonometry, visual field, visual acuity)

### Example 4: The clinical examination template by the General Practitioner (GP) in charge of the health check

This template provides a comprehensive approach to document the key findings and personal history relevant to the patient's current health status and medical background, which will be used to synthesize the overall health check results. This synthesis will be based the patient's questionnaires' results validated by the GP, and the clinical examination.

This detailed information provides a framework automatically pre-populated for the GP to record findings and integrate test results, ensuring a comprehensive overview of the patient's health status during the check-up.

### 1. Personal History

### The personal history will be directly implemented from the questionnaire, however the GP can modified and complete if necessary

- Medical: Medical history
- Surgical: Type of surgery, laterality, year.
- Gynecological: Specific history and findings
- Urological: Specific history and findings.
- Allergy: Details of any allergies.
- Treatment: Current treatments, if any.
- HEMOCULT: Yes/No (a test for hidden blood in the stool).
- Endoscopy: Yes/No - if yes, details of the last endoscopy, normal/abnormal findings, and the year it was performed.
- Vaccinations: Current vaccination status.
- Family History: Cardiovascular Diseases including heart attack/stroke, hypertriglyceridemia, diabetes, high blood pressure, relationship, type of event, age at event.
- Other illnesses such as Alzheimer's, glaucoma, etc.
- Lifestyle: Age, family situation/children, place of residence, profession, smoking (extracted from the questionnaire), number of packs per year, alcohol consumption (number of glasses per week), other (illicit) drugs, diet, physical activity (implemented by the questionnaire with free text for reporting).

### 2. Clinical examination

All data accumulated during the health check will automatically populate this synthesis. Clinical exam should be completed by the GP
- Hemodynamic constants for the day: Blood pressure, heart rate (bpm), oxygen saturation (%), temperature (°C).
- Biometric data: Height (cm), weight (kg), BMI (kg/m^2), abdominal circumference (cm), neck circumference (cm).
- Cardiovascular Examination: Direct inclusion of normal clinical examination findings in the report.
- No chest pain at rest or during exertion, supple and painless calves, bilaterally and symmetrically perceived pulses, regular heart sounds without audible murmurs, no lower limb edema.
- Pulmonary Examination: Clear auscultation with bilateral and symmetrical vesicular murmur, patient breathing comfortably at rest, no exertional dyspnea, no chronic cough or expectoration.
- If thoracic CT: results of the thoracic CT scan.
- Abdominal and pelvic examination.
- Genitourinary examination.
- ENT (Ear, Nose, Throat) examination.
- Dermatological examination.
- Rheumatological Examination and osteoporosis risk:
Gastroenterology:
- The clinical examination should directly state in the report (and in the observation) that it is normal.
- Abdomen is soft and pain-free.
- Liver is of normal size.
- No ascites present.
- No collateral circulation observed.
Abdominal and pelvis Ultrasound:
- Integrate the FULL report of the abdominal ultrasound (not just the conclusion). Neurological:
- The clinical examination should directly state in the report (and in the observation) that it is normal.
"Neurological examination without particularity"
Urological:
- The clinical examination should directly state in the report (and in the observation) that it is normal.
"No urinary functional signs present"
Gynecological:
- In the electronic medical record (DPI), the gynecology examination section will be defined as follows
- Gynecological history:
- GXPX
- Mammography: Yes/No, Year:
- Pap test: Yes/No, Year:
- Plus free text for additional gynecological history

3. Biological:
- The complete blood count is normal.
- The metabolic panel including fasting glucose, total cholesterol, HDL cholesterol (good cholesterol), LDL (bad cholesterol), triglycerides is [...] (specific results to be filled in).
- Glycated hemoglobin is normal at [...] %.
- Calcium level is at [...] mmol/l.
- Thyroid function is normal with a TSH at [...] mUI/l.
- Renal and hepatic function is normal.
- Urinalysis is normal.
- Explore the possibility of directly implementing the results in the yellow highlighted sections and presenting all detailed biology as an annex.

### Example 5: List of medical tests

| General Theme | Type | Name of Test | Description and Objective |
|---|---|---|---|
| Acid-Base Balance | Blood | Anion Gap | Helps determine the cause of acidosis in body fluids |
| Bone Health | Blood | Vitamin D Levels | Crucial for bone health, measured to guide treatment for preventing or treating osteoporosis. |
| Bone Health | Blood | Calcium | Primary mineral in bones, with levels indicating bone health or parathyroid activity. |
| Bone Health | Blood | Phosphorus | Related to bone health and dietary intake, with abnormalities indicating various disorders. |
| Electrolyte Balance | Blood | Sodium, Potassium, Chloride, CO2 | Electrolytes that help maintain fluid balance, nerve and muscle function. |
| Enzyme Activity | Blood | Magnesium | Activates enzymes, with abnormalities indicating potential abuse or metabolic disorders. |
| General Health | Blood | Complete Blood Count | Assesses overall health and detects a range of disorders, including anemia and infection. |
| General Health | Blood | Chemistry Profile | Evaluates proteins, electrolytes, and enzymes to assess organ function. |
| General Health | Blood | Lipid Profile | Measures cholesterol levels to assess cardiovascular risk. |
| Glucose Metabolism | Blood | Glucose | Indicates blood sugar levels, affected by diet, medication, and medical conditions like diabetes. |
| Glucose Metabolism | Blood | Hemoglobin A1C (Glycosylated Hemoglobin) | Shows average blood sugar over three months, indicating diabetes control and risk. |
| Inflammation | Blood | High Sensitivity-CRP (hs-CRP) | Measures the level of CRP, an indicator of inflammation in the body, associated with cardiovascular risk. |
| Kidney Function | Blood | Blood Urea Nitrogen (BUN) / Creatinine | Assesses kidney efficiency in waste excretion, with BUN levels sensitive to hydration status. |
| Liver Function | Blood | Gamma-Glutamyltransferase (GGT) | Enzyme that indicates liver health, affected by alcohol intake, cholesterol levels, and weight. |
| Liver Function | Blood | Alanine Aminotransferase (ALT) / Aspartate Aminotransferase (AST) | Enzymes indicating liver and muscle health, with damage potentially elevating levels. |
| Liver Function | Blood | Alkaline Phosphatase | Related to liver or bone health, indicates bile duct blockage or bone disorders. |
| Liver Function | Blood | ALT/AST | Liver and muscle enzymes, indicating tissue damage. |
| Liver Function | Blood | Bilirubin Total | Levels of bile pigment in the blood, indicating liver function and red blood cell turnover. |
| Lung Health | Blood | Spirometry | Measures lung function to identify respiratory conditions. |
| Men's Health | Blood | PSA Test | Measures a protein produced by the prostate gland to screen for prostate cancer. |
| Men's Health | Blood | Testosterone Levels | Assesses testosterone for its effects on various body functions. |
| Metabolism | Blood | Uric Acid | Byproduct of nucleic acid metabolism, commonly elevated in gout or kidney disorders. |
| Metabolism | Blood | Lactate Dehydrogenase (LD or LDH) | Enzyme related to tissue damage and red blood cell turnover, with isoenzymes pinpointing damage location. |
| Nutritional Health | Blood | Iron/TIBC | Evaluates iron metabolism and storage, indicating anemia or overload. |
| Nutritional Health | Blood | Vitamin B12 | Essential for cell metabolism, nervous system function, and blood cell production. |
| Thyroid Function | Blood | Free T4 | Indicates thyroid hormone levels, assessing thyroid gland activity. |
| Thyroid Health | Blood | TSH | Regulates thyroid hormone production, indicating thyroid function. |
| Vitamin Levels | Blood | Vitamin B12 (Cobalamin) | Essential for cell metabolism, with deficiencies affecting the nervous system and blood cell production. |
| Cardiovascula r | Electrophysiolog y | Exercise Treadmill Test | Assesses heart function and risk of coronary artery disease. |
| Digestive Health | Endoscopy | Colonoscopy | Visual examination of the colon to detect polyps or cancer. |
| Visual Health | Eye | Visual test | Captures images of the retina to detect eye diseases. |
| Visual Health | Eye | Tonometry | Measures intraocular pressure to screen for glaucoma. |
| Digestive Health | Feces | Haemocult | Visual examination of the colon to detect polyps or cancer. |
| Women's Health | Gyne exam | Pap Test | Screens for cervical cancer and other cervical abnormalities. |
| Women's Health | Gyne exam | HPV Screen | Detects human papillomavirus, which is associated with cervical cancer. |
| Bone Health | Imaging | Bone Mineral Densitometry | Assesses bone density to predict fracture risk and diagnose osteoporosis. |
| Cardiovascula r | Ultrasound | Non-Invasive Vascular Screening | Includes tests like Carotid Ultrasound, Abdominal Aorta Ultrasound, and Ankle-Brachial Index to evaluate blood flow and risk for vascular diseases. |
| Kidney Function | Urine | Urinary Microalbumin | Sensitive measure of protein leakage from kidneys, indicating early kidney disease or cardiovascular risk. |
| Kidney Function | Urine | Urinary Microalbumin/Creatinin e Ratio | Screens for early kidney disease, particularly in diabetes. |
| Women's Health | X-Ray | Mammography | X-ray of the breasts to detect abnormalities. |

### Example 6: Questionnaires

### General questionnaire

| Q | Theme | Question Type | Question | Response Choices |
|---|---|---|---|---|
| 2 | Demographic Data | Open | Last name | - |
| 3 | Demographic Data | Open | First name | - |
| 4 | Demographic Data | Single Choice | Sex | Female, Male |
| 5 | Demographic Data | Number (Open) | What is your height? (cm) | - |
| 6 | Demographic Data | Number (Open) | Please enter your weight (Kg) | - |
| 7 | Demographic Data | Date (Autocomplete) | Date of birth | - |
| 8 | Demographic Data | Open | Personal email | - |
| 9 | Demographic Data | Open | Telephone number to reach you | - |
| 10 | Demographic Data | Open | Country of Residence | - |
| 11 | Demographic Data | Open | Postal code | - |
| 12 | Demographic Data | Closed (Yes/No) | Do you have a treating doctor (GP)? | YES, NO |
| 13 | Demographic Data | Open | Who is your treating doctor? | - |
| 14 | Demographic Data | Open | Can you give us their address? | - |
| 15 | Demographic Data | Closed (Yes/No) | Would you like the report of this health check to be sent to your attending physician? | YES, NO |
| 16 | Personal Situation | Multiple Choice | Marital status | Married, in a civil partnership, or in a stable union, In a lasting relationship, but not living together, Single, Divorcee, Widower, Do not wish to respond |
| 17 | Education | Multiple Choice | What is the highest level of education you have achieved? | I left school before the age of 16, I attended secondary school (high school), but did not graduate, Secondary education graduate (Bac or High School Diploma), License (Bachelor), 2nd (Master) and 3rd cycle (Doctorate) university diploma and professional diplomas (doctors, pharmacist, veterinarian, engineer, lawyers, etc.) |
| 18 | Professional Status | Multiple Choice | Professional categories? | Detailed list of professional categories and statuses |
| 19 | Demographic Data | Open | Specify | - |
| | | | | |
| 20 | Family History | Closed | Are there any conditions or illnesses that affect your family? | YES, NO |
| 21 | Family History | Open | If yes, specify | - |
| 22 | Family History | Closed | Cardiovascular - Have any of your close relatives suffered from various cardiovascular diseases? | YES, NO |
| 23 | Family History | Table | If YES, please carefully complete this table regarding your family cardiovascular history | Mother, Father, Brother or sister, High blood pressure, Diabetes, Heart disease - Heart attack, Cerebral Vascular Accident (CVA) |
| 24 | Family History | Closed | Did any of your close relatives suffer a heart attack or stroke before the age of 60? | YES, NO |
| 25 | Family History | Table | If YES, please complete this table for family history of cardiovascular disease before age 60 | Mother, Father, Brother or sister, Heart disease - Heart attack, Cerebral Vascular Accident (CVA) |
| 26 | Family History | Closed | Cancer (excluding specific female cancer) - Have any of your close relatives suffered from one of these cancers? | YES, NO |
| 27 | Family History | Table | If yes, specify | Father, Mother, Brother or sister, Colorectal and digestive cancer, Pancreatic cancer, Malignant melanoma (skin cancer), Kidney cancer |
| 28 | Family History | Closed | Did any of your close relatives suffer from colorectal cancer before the age of 55? | YES, NO |
| 29 | Family History | Closed | Are you a woman? | YES, NO |
| 30 | Family History | Table | Have any of your close relatives had breast or ovarian cancer? | Mother, Sister(s), Breast cancer, Ovarian cancer before age 50, Ovarian cancer |
| 31 | Family History | Closed (Men) | Did any of your close relatives have prostate cancer before the age of 60? | YES, NO |
| 32 | Family History | Closed (Men) | Have you ever done a PSA test? | YES, NO |
| 33 | Family History | Open | If yes, please indicate the date and dosage of the last dosage | Date, Results |
| | COMMENTS | | | |
| 34 | Personal Health | Closed | Have you been hospitalized for at least one night in the last five years? | YES, NO |
| 35 | Personal Health | Open | If YES, specify | - |
| 36 | Personal Health | Closed | Have you lost a significant amount of weight unintentionally in the past 24 months? | YES, NO |
| 37 | Personal Health | Open | If yes, specify the number of kg lost | - |
| 38 | Personal Health | Multiple Choice | Has a doctor told you that you have any of the following conditions or have you had any of the following procedures? | Glaucoma, Cataract, High cholesterol, Asthma, Emphysema/chronic bronchitis, Kidney/bladder stones, etc. |
| 39 | Personal Health | Closed | Have you ever undergone any surgical procedure(s)? | YES, NO |
| 40 | Personal Health | Open | If yes, specify | - |
| 41 | Cardiovascular | Closed | Has a doctor ever told you that you have heart problems, blood circulation problems, or blood clots (thrombosis)? | YES, NO |
| 42 | Cardiovascular | Multiple Choice | Please check below the conditions or procedures that your doctors believe have affected you. | Cardiac arrest, Heart failure, Coronary angiography, Coronary bypass, Coronary artery angioplasty, Carotid surgery, etc. |
| 43 | General | Closed | During the last 12 months, have you had a serious illness or lost consciousness? | YES, NO |
| 44 | Cardiovascular | Closed | Has a doctor ever told you that you have high blood pressure? | YES, NO |
| 45 | Cardiovascular | Multiple Choice | How old were you when you were told you had high blood pressure? | Under 30, 30-39, 40-49, 50-59, 60-69, Over 70 years old |
| 46 | Cardiovascular | Closed | Are you taking or have you ever taken medication for high blood pressure? | YES, NO |
| 47 | Cardiovascular | Closed | Has a doctor ever told you that you have angina? | YES, NO |
| 48 | Cardiovascular | Closed | Are you taking medication for angina? Or other heart medication | YES, NO |
| 49 | Cardiovascular | Closed | Has a doctor ever told you that you have peripheral arterial disease? | YES, NO |
| 50 | Cardiovascular | Closed | If YES, have you ever had surgery, angioplasty, or a stent to improve circulation in your legs? | YES, NO |
| 51 | Neurological History | Closed | Do you have, or have you ever had, significant memory problems? | YES, NO |
| 52 | Neurological History | Closed | Have you ever had a stroke, mini-stroke or transient ischemic attack (TIA)? | YES, NO |
| 53 | Neurological History | Closed | Have you ever had epilepsy or seizures? | YES, NO |
| 54 | Digestive History | Closed | Have you ever had a colonoscopy? | YES, NO |
| 55 | Digestive History | Multiple Choice | If yes, when was the last time you had a colonoscopy? | Less than 5 years old, 5 years or more |
| 56 | Digestive History | Closed | Have you ever had polyps removed from the colon, intestines or rectum? | YES, NO |
| 57 | Digestive History | Closed | Have you ever given a stool sample for colorectal cancer screening? | YES, NO |
| 58 | Cancer History | Closed | Has a doctor ever told you that you have cancer, a tumor, or a malignant tumor? | YES, NO |
| | | Medications and vaccines (1 minute) | | |
| 72 | Medication | Closed | Do you take medication regularly? | YES, NO |
| 73 | Medication | Open | If yes, list these medications | - |
| 74 | Women's Health | Closed | For women: Do you take oral contraception (pill)? | YES, NO |
| 75 | Vaccination | Multiple Choice | Please provide as much information as possible regarding updating your vaccinations. | YES, NO, Do not know; Specific vaccines: DTP, Flu, Covid-19, Shingles, Pneumococcus |
| | | | | |
| 76 | Lifestyle - Physical | Open (Numerical) | In the past 7 days, how many days were there that you did intense physical activities such as carrying heavy loads, mountain biking, running, or playing soccer? | 0 - I did not have any intense physical activity, 1, 2, 3, 4, 5, 6, 7 |
| 77 | Lifestyle - Physical | Open (Numerical) | When you did intense physical activities on one of these days, how much time did you spend on average per day? (in minutes) | (User to provide number of minutes) |
| 78 | Lifestyle - Physical | Open (Numerical) | During the last 7 days, how many days were there when you did moderate physical activities such as carrying light loads, cycling leisurely, swimming, etc.? Do not include walking. | 0 - I did not do moderate physical activities, 1, 2, 3, 4,5,6,7 |
| 79 | Lifestyle - Physical | Open (Numerical) | When you did moderate activities on one of these days, how much time did you spend on average per day? (in minutes) | (User to provide number of minutes) |
| 80 | Lifestyle - Physical | Open (Numerical) | In the last 7 days, how many days were there when you walked for at least 10 minutes straight? | 0 - I haven't walked for more than 10 minutes at a time, 1, 2, 3, 4, 5, 6, 7 |
| 81 | Lifestyle - Physical | Open (Numerical) | When you walked on one of these days, how long did you spend on average? (in minutes) | (User to provide number of minutes) |
| 82 | Lifestyle - Physical | Closed | Do you use an application that measures the number of steps taken per day on your smartphone? | Yes, No |
| 83 | Lifestyle - Physical | Open (Numerical) | If YES, report the average number of steps measured per day over the last 7 days? | (User to provide average number of steps) |
| 84 | Lifestyle - Sedentariness | Open (Numerical) | Over the past 7 days, how much time did you spend sitting on average on a weekday? (in hours) | (User to provide number of hours) |
| 85 | Lifestyle - Alcohol | Multiple Choice | How often do you consume alcoholic beverages? | Never or almost never, At least once a month, At least two to four times a month, At least two to three times a week, Four times a week or more |
| 86 | Lifestyle - Alcohol | Multiple Choice | How many drinks do you consume in a normal drinking day? | 1 or 2, 3 or 4, 5 or 6, 7 to 9, 10 or more |
| 87 | Lifestyle - Alcohol | Closed | Have those around you ever made comments to you about your alcohol consumption? | YES, NO |
| 88 | Lifestyle - Alcohol | Closed | Do you find yourself drinking alcohol in the morning to feel good? | YES, NO |
| 89 | Lifestyle - Alcohol | Closed | Have you ever had a drink or more and not remembered what you said or did the next morning? | YES, NO |
| 90 | Lifestyle - Tobacco | Multiple Choice | Tobacco consumption | I have never smoked, I am a smoker, I used to smoke but I stopped |
| 91 | Lifestyle - Tobacco | Open | When did you stop smoking? Approximate date | (User to provide date) |
| Lifestyle - Tobacco | Multiple Choice | How much do you smoke (or did you smoke) on average per day? | 1 to 3, 3 to 5, 5 to 10, 10 to 20, 20 to 30, More than 30 | |
| 94 | Lifestyle - Tobacco | Closed | Do you smoke cannabis? | YES, NO |
| 95 | Lifestyle - Tobacco | Closed | Passive smoker - I didn't smoke but I was exposed to a heavy smoking environment for a long time. Several years | YES, NO |

### Sress-Anxiety-Moud questionnaire

| GAD7 | | | | |
|---|---|---|---|---|
| Question Number | Theme | Question Type | Question (Paraphrased) | Response Choices |
| 1 | General Anxiety | Scale | Frequency of feeling nervous, anxious, or on edge | 0-Not at all; 1-several day; 2-more tan half trhe days; 3-Nearly every day) |
| 2 | Worry | Scale | Ability to control worrying | 0-Not at all; 1-several day; 2-more tan half trhe days; 3-Nearly every day) |
| 3 | Worry | Scale | Frequency of worrying about different things | 0-Not at all; 1-several day; 2-more tan half trhe days; 3-Nearly every day) |
| 4 | Relaxation | Scale | Difficulty relaxing | 0-Not at all; 1-several day; 2-more tan half trhe days; 3-Nearly every day) |
| 5 | Restlessness | Scale | Being so restless it's hard to sit still | 0-Not at all; 1-several day; 2-more tan half trhe days; 3-Nearly every day) |
| 6 | Irritability | Scale | Becoming easily annoyed or irritable | 0-Not at all; 1-several day; 2-more tan half trhe days; 3-Nearly every day) |
| 7 | Fear | Scale | Feeling afraid as if something awful might happen | 0-Not at all; 1-several day; 2-more tan half trhe days; 3-Nearly every day) |

| PHQ9 | | | | |
|---|---|---|---|---|
| Question Number | Theme | Question Type | Question (Paraphrased) | Response Choices |
| 1 | Interest | Scale | Interest level in doing activities | 0-Not at all; 1-several day; 2-more tan half trhe days; 3-Nearly every day) |
| 2 | Mood | Scale | Frequency of feeling down, depressed, or hopeless | 0-Not at all; 1-several day; 2-more tan half trhe days; 3-Nearly every day) |
| 3 | Sleep | Scale | Difficulty with sleep, either too much or too little | 0-Not at all; 1-several day; 2-more tan half trhe days; 3-Nearly every day) |
| 4 | Energy | Scale | Levels of tiredness or having little energy | 0-Not at all; 1-several day; 2-more tan half trhe days; 3-Nearly every day) |
| 5 | Eating Habits | Scale | Problems with poor appetite or overeating | 0-Not at all; 1-several day; 2-more tan half trhe days; 3-Nearly every day) |
| 6 | Self-Image | Scale | Feelings about self, including perceived failure | 0-Not at all; 1-several day; 2-more tan half trhe days; 3-Nearly every day) |
| 7 | Concentration | Scale | Trouble with concentration on activities | 0-Not at all; 1-several day; 2-more tan half trhe days; 3-Nearly every day) |
| 8 | Motor Function | Scale | Observably altered motor functions, such as moving slowly | 0-Not at all; 1-several day; 2-more tan half trhe days; 3-Nearly every day) |
| 9 | Suicidal Ideation | Scale | Thoughts of self-harm or being better off dead | 0-Not at all; 1-several day; 2-more tan half trhe days; 3-Nearly every day) |

### Osteoporosis questionnaire

| Q | Question Type | Question | Response Choices |
|---|---|---|---|
| 1 | Closed, Single choice | Was osteoporosis diagnosed in your father or mother, or did one of them fracture their femoral neck following a minor shock or fall? Yes/No. | YES/NO |
| 2 | Closed, Single choice | Is one of your parents stooped? Yes/No. | YES/NO |
| 3 | Closed, Single choice | Have you fractured a bone following a minor shock or fall? Yes/No. | YES/NO |
| 4 | Closed, Single choice | Do you frequently fall (more than once a year) or fear falling because you feel frail? Yes/No. | YES/NO |
| 5 | Closed, Single choice | Has your height decreased by more than 3 cm after the age of 40? Yes/No. | YES/NO |
| 6 | Closed, Single choice | Have you taken corticosteroids (cortisone, prednisone, etc.) for more than 3 consecutive months? Yes/No. | YES/NO |

### Women - gynecology questionnaire

| Q | Theme | Question Type | Question | Response Choices |
|---|---|---|---|---|
| 1 | General Information | Open | What is your age? | (Patient to fill in age) |
| 2 | Menstrual History | Open | Age at first menstrual period | (Patient to fill in age) |
| 3 | Obstetric History | Open/Close | Do you have children? | Yes / No |
| 4 | Obstetric History | Numerical if yes to Q3 | If yes, how many? | (Patient to fill in number) |
| 5 | Obstetric History | Numerical if yes to Q3 | Age at first childbirth | (Patient to fill in age) |
| 6 | Obstetric History | Numerical | Number of vaginal deliveries | (Patient to fill in number) |
| 7 | Obstetric History | Numerical | Number of cesarean sections | (Patient to fill in number) |
| 8 | Obstetric History | Numerical | Number of miscarriages | (Patient to fill in number) |
| 9 | Obstetric History | Close | Have you ever had fertility treatment? | Yes / No |
| 10 | Breastfeeding | Close | Have you breastfed? | Yes / No |
| 11 | Pregnancy Pathologies | Close | Did you have any of these conditions during pregnancy? | HELLP syndrome, High blood pressure, Preeclampsia, Intrauterine growth restriction, Preterm labor < 35 weeks, Gestational diabetes |
| 12 | Menopausal Status | Close | Are you menopausal? | Yes / No |
| 13 | Menopause Details | Open if yes to Q12 | If yes, duration of amenorrhea | (Patient to fill in duration) |
| 14 | Menopause Details | Close if yes to Q12 | If menopausal, did it start before age 45? | Yes / No |
| 15 | Menopause Symptoms | Multiple Choice if yes to Q12 | If yes, current symptomatology | Number of hot flashes per day, Night sweats, Mood disturbances, Memory troubles, Headaches/mig raines, Joint pains, etc. |
| 16 | Menstrual Regularity | Close if no to Q12 | If not menopausal, are your cycles regular? | Yes / No |
| 17 | Menstrual Interruption | Close if no to Q12 | Have your periods stopped for 12 consecutive months or more for reasons other than pregnancy, menopause, or hysterectomy? | Yes / No |
| 18 | Contraception | Open/Close if no to Q12 | If not menopausal, do you take contraception? If yes, which type? | Pill (estrogen-progestin), IUD |
| 19 | Family Gynecological History | Close | Family history of breast cancer (grandparents/parent s/sister)? | Yes / No |
| 20 | Family Genetic History | Close if yes to Q19 | If yes, is there a BRCA or PALB 2 mutation in the family? | Yes / No |
| 21 | Family Gynecological History | Close | Any other gynecological cancer in the family (grandparents/parent s/sister)? | (Patient to fill in relation and type of cancer) |
| 22 | Personal Gynecological History | Multiple Choice | Have you been diagnosed with any of these non-cancerous pathologies? | Endometriosis, Polycystic ovary syndrome (PCOS), Pelvic floor insufficiency |
| 23 | HPV Diagnosis | Close | Have you been diagnosed with HPV? | Yes / No |
| 24 | HPV Treatment | Close if yes to Q23 | If yes, have you undergone a conization? | Yes / No |
| 25 | HPV Vaccination | Close | Are you vaccinated against HPV? | Yes / No |
| 26 | Pap Smear | Close | Have you ever had a Pap smear? | Yes / No |
| 27 | Pap Smear | Open if yes to Q26 | If yes, what is the date of your last Pap smear? | (Patient to fill in date) |
| 28 | Mammography | Close | Have you ever had a mammogram? | Yes / No |
| 29 | Mammography | Open if yes to Q28 | If yes, what is the date of your last mammogram? | (Patient to fill in date) |
| 30 | Breast Cancer Diagnosis | Close | Have you been diagnosed with breast cancer? | Yes / No |
| 31 | Breast Cancer Details | Open if yes to Q30 | If yes, what was your age at onset? | (Patient to fill in age) |
| 32 | Genetic Testing | Close if yes to Q30 | If diagnosed, was a mutation search conducted (BRCA)? | Yes / No |
| 33 | Surgical Treatment | Close if yes to Q30 | If diagnosed, did you undergo surgery? | Yes / No |
| 34 | Chemotherapy | Close if yes to Q30 | If diagnosed, did you receive chemotherapy? | Yes / No |
| 35 | Radiotherapy | Close if yes to Q30 | If diagnosed, did you receive radiotherapy? | Yes / No |
| 36 | Hormone Therapy | Close if yes to Q30 | If diagnosed, did you receive hormone therapy? | Yes / No |

### Cardiology questionnaire

| Q | Theme | Question Type | Question | Response Choices |
|---|---|---|---|---|
| 1 | Cardiovascular Symptoms | Closed | Do you experience chest pain or tightness? | Yes / No |
| 2 | Cardiovascular Symptoms | Multiple Choice if yes to Q1 | If yes, when does the pain occur? | During physical effort, At rest, At night |
| 3 | Cardiovascular Symptoms | Multiple Choice if yes to Q1 | Does it appear when it's cold or hot? | Yes / No |
| 4 | Cardiovascular Symptoms | Closed if yes to Q1 | Does it get worse after a heavy meal? | Yes / No |
| 5 | Cardiovascular Symptoms | Closed if yes to Q1 | Is the pain intense enough to make you stop your activity? | Yes / No |
| 6 | Cardiovascular Symptoms | Closed if yes to Q1 | Does the pain go away when you rest? | Yes / No |
| 7 | Cardiovascular Symptoms | Closed if yes to Q1 | Does it recur when resuming activity or increasing intensity? | Yes / No |
| 8 | Physical Capability | Numerical | How many floors can you climb without getting out of breath? | (Number of floors) |
| 9 | Breathlessness | Closed | Do you have unusual shortness of breath? | Yes / No |
| 10 | Breathlessness | Multiple Choice if yes to Q9 | If yes, does it occur during physical activity? | Yes / No |
| 11 | Breathlessness | Closed if yes to Q9 | Do you wake up feeling suffocated? | Yes / No |
| 12 | Breathlessness | Closed if yes to Q9 | Do you cough or wheeze when short of breath? | Yes / No |
| 13 | NYHA Functional Class | Scale if yes to Q9 | Degree of functional impairment (NYHA score): | Stage II, Stage III, Stage IV |
| 14 | Syncope | Closed | Have you experienced loss of consciousness? | Yes / No |
| 15 | Palpitations | Closed | Do you have palpitations? | Yes / No |
| 16 | Sleep Habits | Numerical | How many pillows do you use to sleep? | (Number of pillows) |
| 17 | Leg Pain | Closed | Do you have pain in your lower limbs? | Yes / No |
| 18 | Leg Pain | Closed if yes to Q17 | If yes, is it a cramping pain that occurs during walking and requires you to stop? | Yes / No |
| 19 | Leg Pain | Closed if yes to Q17 | Does the pain disappear within 2 to 3 minutes of rest and reappear upon resuming walking? | Yes / No |
| 20 | Leg Pain | Closed | Do you have heavy legs? | Yes / No |
| 21 | Hemoptysis | Closed | Have you ever coughed up blood? | Yes / No |

### Questionnaire about sleep

| Q | Theme | Question Type | Question | Response Choices |
|---|---|---|---|---|
| 1 | Sleep Difficulty | Closed, single choice | Difficulty falling asleep, staying asleep, or feeling rested in morning | Never, Sometimes, Usually, Always |
| 2 | Unintentional Sleep | Closed, single choice | Falling asleep unintentionally or fighting to stay awake during the day | Never, Sometimes, Usually, Always |
| 3 | Daily Interference | Closed, single choice | Sleep difficulties or daytime sleepiness interfering with daily activities | Never, Sometimes, Usually, Always |
| 4 | Sleep Prevention | Closed, single choice | Work or activities preventing enough sleep | Never, Sometimes, Usually, Always |
| 5 | Snoring | Closed, single choice | Snoring loudly | Never, Sometimes, Usually, Always |
| 6 | Breathing Issues | Closed, single choice | Holding breath, breathing pauses, or stopping breathing in sleep | Never, Sometimes, Usually, Always |
| 7 | Restlessness | Closed, single choice | Restless or "crawling" feelings in legs at night | Never, Sometimes, Usually, Always |
| 8 | Limb Movements | Closed, single choice | Repeated rhythmic leg jerks or twitches during sleep | Never, Sometimes, Usually, Always |
| 9 | Disturbed Sleep | Closed, single choice | Nightmares, screaming, walking, punching, or kicking in sleep | Never, Sometimes, Usually, Always |
| 10 | Sleep Disturbances | Open | Factors disturbing sleep (e.g., worries, pain, medications) | (Open response) |
| 11 | Emotional State | Closed, single choice | Feeling sad or anxious | Never, Sometimes, Usually, Always |
| 12 | Snoring | Closed, single choice | Do you snore loudly (louder than talking or loud enough to be heard through closed doors)? | Yes / No |
| 13 | Snoring Frequency | Closed, single choice | How often do you snore? | Almost every day, 3-4 times a week, 1-2 times a week, Rarely, Never |
| 14 | Breathing Pauses | Closed, single choice | Has anyone observed you stop breathing during your sleep? | Yes / No |
| 15 | Fatigue | Closed, single choice | Do you often feel tired, fatigued, or sleepy during daytime? | Yes / No |
| 16 | Sleepiness | Scale | How likely are you to doze off or fall asleep in the following situations, in contrast to feeling tired? | (Rating for each situation, such as sitting and reading, watching TV) |

### Questionnaire about diet

| Q | Theme | Question Type | Question | Response Choices |
|---|---|---|---|---|
| 1 | Eating Habits | Closed, Single choice | Do you skip breakfast more than once a week? | Yes / No |
| 2 | Eating Habits | Closed, Single choice | Do you skip lunch more than once a week? | Yes / No |
| 3 | Eating Habits | Closed, Single choice | Do you skip evening meals more than once a week? | Yes / No |
| 4 | Eating Habits | Closed, Single choice | Do you skip meals and snack instead on most days? | Yes / No |
| 5 | Fruit and Vegetables | Closed, Single choice | Do you eat more than 5 portions of fruit and/or vegetables every day? | Yes / No |
| 6 | Fruit and Vegetables | Closed, Single choice | Do you eat more than 4 different varieties of fruit each week? | Yes / No |
| 7 | Fruit and Vegetables | Closed, Single choice | Do you eat more than 4 different varieties of vegetables each week? | Yes / No |
| 8 | Fat | Closed, Single choice | Do you choose low-fat products when available? | Yes / No |
| 9 | Fat | Closed, Single choice | Do you choose baked, steamed, or grilled options when available rather than fried foods? | Yes / No |
| 10 | Fat | Closed, Single choice | Do you opt for lean cuts of meat or remove visible fat, like skin on chicken or bacon rind? | Yes / No |
| 11 | Fat | Closed, Single choice | Did you eat any oily fish last week, like salmon, mackerel, or sardines? | Yes / No |
| 12 | Fat | Closed, Single choice | Do you include some unsalted nuts and seeds in your diet? | Yes / No |
| 13 | Starchy Foods | Closed, Single choice | Do you base your main meals around starchy foods, like potatoes, pasta, rice, or bread? | Yes / No |
| 14 | Starchy Foods | Closed, Single choice | Do you regularly choose wholemeal bread or rolls rather than white? | Yes / No |
| 15 | Starchy Foods | Closed, Single choice | Do you regularly eat wholegrain cereals with no added sugar? | Yes / No |
| 16 | Starchy Foods | Closed, Single choice | Do you regularly include pulses, like beans and lentils, in your diet? | Yes / No |
| 17 | Sugar | Closed, Single choice | Do you regularly eat sugar-coated breakfast cereals or add sugar to your breakfast cereals? | Yes / No |
| 18 | Sugar | Closed, Single choice | Do you add sugar to your drinks? | Yes / No |
| 19 | Sugar | Closed, Single choice | Do you regularly drink sweet fizzy drinks? | Yes / No |
| 20 | Sugar | Closed, Single choice | Do you regularly eat cakes, sweets, chocolate, or biscuits at work? | Yes / No |
| 21 | Salt | Closed, Single choice | Do you regularly add salt to food during cooking? | Yes / No |
| 22 | Salt | Closed, Single choice | Do you regularly add salt to meals at the table? | Yes / No |
| 23 | Salt | Closed, Single choice | Do you regularly eat savoury snacks at work, like crisps or salted nuts? | Yes / No |
| 24 | Salt | Closed, Single choice | Do you regularly eat pre-prepared meals, like sandwiches, ready meals, or canned soups? | Yes / No |
| 25 | Salt | Closed, Single choice | Do you regularly eat processed meats, like ham or bacon, or smoked fish? | Yes / No |
| 26 | Salt | Closed, Single choice | Has your GP advised you that you have high blood pressure? | Yes / No |
| 27 | Drinks and Alcohol | Closed, Single choice | Do you drink plenty of fluids at regular intervals during the working day? | Yes / No |
| 28 | Drinks and Alcohol | Closed, Single choice | Do you opt for a variety of different drinks including water at work? | Yes / No |
| 29 | Drinks and Alcohol | Closed, Single choice | Do you avoid sugary fizzy drinks? | Yes / No |

### Questionnaire about global physical activity

| Q | Theme | Question Type | Question | Response Choices |
|---|---|---|---|---|
| 1 | Work-Related Activity | Closed | Does your work involve vigorous-intensity activity like heavy lifting for at least 10 minutes continuously? | Yes / No |
| 2 | Work-Related Activity | Numerica l | On how many days do you do vigorous-intensity activities as part of your work? | (Number of days) |
| 3 | Work-Related Activity | Numerica l | How much time do you spend doing vigorous-intensity activities at work on a typical day? | (Hours and minutes) |
| 4 | Work-Related Activity | Closed | Does your work involve moderate-intensity activity like brisk walking for at least 10 minutes continuously? | Yes / No |
| 5 | Work-Related Activity | Numerica l | On how many days do you do moderate-intensity activities as part of your work? | (Number of days) |
| 6 | Work-Related Activity | Numerica l | How much time do you spend doing moderate-intensity activities at work on a typical day? | (Hours and minutes) |
| 7 | Travel-Related Activity | Closed | Do you walk or use a bicycle for at least 10 minutes continuously to get to and from places? | Yes / No |
| 8 | Travel-Related Activity | Numerica l | On how many days do you walk or bicycle to get to and from places? | (Number of days) |
| 9 | Travel-Related Activity | Numerica l | How much time do you spend walking or bicycling for travel on a typical day? | (Hours and minutes) |
| 10 | Recreation al Activity | Closed | Do you do any vigorous-intensity sports or recreational activities for at least 10 minutes continuously? | Yes / No |
| 11 | Recreation al Activity | Numerica l | On how many days do you do vigorous-intensity sports or recreational activities? | (Number of days) |
| 12 | Recreation al Activity | Numerica l | How much time do you spend doing vigorous-intensity sports or recreational activities on a typical day? | (Hours and minutes) |
| 13 | Recreation al Activity | Closed | Do you do any moderate-intensity sports or recreational activities for at least 10 minutes continuously? | Yes / No |
| 14 | Recreation al Activity | Numerica l | On how many days do you do moderate-intensity sports or recreational activities? | (Number of days) |
| 15 | Recreation al Activity | Numerica l | How much time do you spend doing moderate-intensity sports or recreational activities on a typical day? | (Hours and minutes) |
| 16 | Sedentary Behavior | Numerica l | How much time do you usually spend sitting or reclining on a typical day? | (Hours and minutes) |

### Example 7: Health Check

| CHECK UP | General assesment | Cardiovascular assesment | Gynecological assesment | Smokers assesment | NASH assesment | Women's risk assesment |
|---|---|---|---|---|---|---|
| CONSULTATIONS | | | | | | |
| General practitioner | * | | | | | |
| Cardiologist | | * | | | | |
| Gynecologist | | | * | | | |
| Cancerologist | | | | | | * |
| ENT | | | | * | | |
| Dietician | * | | | | * | |
| Radiologist | | | | | | * |
| Hepato-gastro-enterologist | | | | | * | |

| EXAMS | | | | | | |
|---|---|---|---|---|---|---|
| *Complete blood test* | | | | | | |
| Serology (hepatitis B + hepatitis C) | * | | | | | |
| HIV screening | * | | | | | |
| Protein electrophoresis (after age 60) | * | | | | | |
| Syphilis | * | | | | | |
| Doping (vitamin D) | * | | | | | |
| CBC platelets (sedimentation rate, CRP) | * | | | | | |
| Metabolic profile: total cholesterol, HDL cholesterol, LDL cholesterol, triglycerides | * | | | | | |
| triglycerides, glycemia HbA1c, uric acid, calcemia | * | | | | | |
| Renal function (natremia, kalemia, creatinine, protidemia) | * | | | | | |
| Liver function (ASAT-ALAT, GAMMA GT, alkaline phosphatases) | * | | | | | |
| Thyroid function (TSH) | * | | | | | |
| Ferritin | * | | | | | |

| Men | | | | | | |
|---|---|---|---|---|---|---|
| PSA (prostate-specific antigen) from age 45 | * | | | | | |

| Urine | | | | | | |
|---|---|---|---|---|---|---|
| Sugar, albumin, cytology | * | | | | | |
| Microalbuminuria | | * | | | | |
| Urine smear | | | | * | | |

| OC-Sensor immunoassay | | | | | | |
|---|---|---|---|---|---|---|
| Blood stool (1 day) colon cancer screening | * | | | | | |

| *Biological assesment* | | | | | | |
|---|---|---|---|---|---|---|
| Fibro testing | | | | | * | |
| Steato testing | | | | | * | |
| Nash testing | | | | | * | |

| *Medical Imaging* | | | | | | |
|---|---|---|---|---|---|---|
| Elasto MRI | | | | | * | |
| Cardiac MRI | | | | | * | |

| *Medico-technical examinations* | | | | | | |
|---|---|---|---|---|---|---|
| Impedance measurement (height, weight) | * | | | | | |
| DNA sampling on a saliva sample | | | | | | * |
| Abdominal and pelvic ultrasound | * | | | | | |

| Vision | | | | | | |
|---|---|---|---|---|---|---|
| Color | * | | | | | |
| Optometry | * | | | | | |
| Tonometry (glaucoma test) | * | | | | | |

| Hearing | | | | | | |
|---|---|---|---|---|---|---|
| Audiometry | * | | | | | |

| Lungs | | | | | | |
|---|---|---|---|---|---|---|
| Low Dose Lung Scanner | | | | * | | |
| Respiratory Functional Investigation | * | | | | | |
| Lung radiology (on medical advice) | * | | | | | |

| Cardiac | | | | | | |
|---|---|---|---|---|---|---|
| Moritoring of arterial pressure (if indicated to detect hypertension) | | * | | | | |
| SPI: systolic pressure index (measurement of arm and ankle blood pressure) | | * | | | | |
| Echodoppler of the supra-aortic trunks | | * | | | | |
| Cardiac Doppler ultrasound | | * | | | | |
| Coronary calcium score by CT scan | | * | | | | |
| Indirect calculation of Vo2Max | | * | | | | |
| Cardiac ultrasound | | * | | | | |
| Resting/effort electrocardiogram | * | | | | | |
| Holter ECG | * | | | | | |
| Sudoscan | * | | | | | |
| Holter blood pressure | * | * | | | | |
| Ventilatory polygraph | * | * | | | | |

| Vaccinations | | | | | | |
|---|---|---|---|---|---|---|
| DTPCOP (if indicated) | * | | | | | |

| Women's exams | | | | | | |
|---|---|---|---|---|---|---|
| Pap smear + HPV test | | | * | | | |
| Digital mammography with tomosynthesis | | | * | | | * |
| Breast ultrasound | | | * | | | * |

| ENT | | | | | | |
|---|---|---|---|---|---|---|
| Naso-fibroscopy | | | | * | | |

| Questionnaire | | | | | | |
|---|---|---|---|---|---|---|
| Stress assessment using the STIMULUS questionnaire (stress assessment) | * | | | | | |
| Personal and family data questionnaire | | | | | | * |
| Preliminary medical questionnaire | | | | | * | |

### Example 8 : Hybrid approach (see Figure 9)

This approach aims to utilize the strengths of both traditional epidemiological knowledge and modern machine learning techniques to provide a comprehensive, accurate, and personalized health risk assessment. It acknowledges the value of existing scientific data while also recognizing the potential of AI to uncover new insights from emerging data trends. The ultimate goal is to enable proactive and tailored health management strategies for individuals.

The type of model will depend on the nature of data:
Continuous and Categorical Data: Biostatistic approach (Linear and logistic regression); Support Vector Machines (SVMs); Random Forests and Boosted Decision Trees (like XGBoost, LightGBM); Deep Neural Networks: For data with complex structures or nonlinear interactions, neural networks can capture complex relationships between variables. Specific architectures like Convolutional Neural Networks (CNNs) for image analysis and Recurrent Neural Networks (RNNs) for sequential data can be particularly useful.
Temporal and time sequence: RNNs and Variants (LSTM, GRU); Classical Time-Series Models (like ARIMA, SARIMA); Transformers and Attention Models:
   For spatial data (image): Convolutional Neural Networks (CNNs) and 3D Convolutional Neural Networks.

### Example 9:

Risk prediction models will use a subset of tests and factors that are most relevant and have the highest predictive value for the disease in question. This approach balances the need for accurate risk assessment with practical considerations such as cost, patient burden, and the availability of tests.

Risk prediction models typically identify key risk factors and biomarkers that can be used to estimate the likelihood of a disease developing. These models are based on clinical research that determines which factors are most strongly associated with the disease.

### Predicting the Risk of Type 2 Diabetes

For Type 2 diabetes, not all potential tests need to be conducted to assess risk. A commonly used risk prediction model is the Finnish Diabetes Risk Score (FINDRISC), which uses the following factors:
1. Age: Older age increases the risk.
2. BMI: Higher body mass index is a risk factor.
3. Waist circumference: Abdominal obesity is a strong risk factor.
4. Physical activity: Lack of regular physical activity increases risk.
5. Consumption of fruits and vegetables: Low consumption is associated with higher risk.
6. Medication for high blood pressure: Indicates cardiovascular issues which are related to diabetes risk.
7. History of high blood glucose: Points to potential pre-diabetes.
8. Family history of diabetes: A genetic predisposition can increase risk.

These factors can be self-reported or measured with a few simple tests, and do not require comprehensive testing for an initial risk assessment. However, if the risk score indicates a high risk of diabetes, a healthcare provider may recommend additional tests, such as blood glucose levels or HbA1c, and insulin resistance for a more definitive assessment. These risk assessments can be updated in real-time as new data becomes available, whether from regular health monitoring or specific diagnostic tests. The system can alert the healthcare provider if the patient's risk level crosses a certain threshold, prompting further investigation or intervention.

This approach allows for efficient and focused health monitoring, targeting the most informative and cost-effective methods to assess disease risk.

### Example 10:

An interface with a medical device can be used to incorporate lab test results into a patient's database and make them accessible via a mobile app, using interoperability standards like HL7 or FHIR (Fast Healthcare Interoperability Resources):
Practical Scenario: Biology Test Results Integration
Test Conducted and Data Generated: The patient undergoes a blood test, the automat generates the results, which include various biomarkers like glucose levels, cholesterol, hemoglobin, etc.
Data Encoding and Transmission: Data are encoded using an interoperability standard (such as FHIR), which structures the data into a universally understandable format. The structured data is then transmitted securely to the patient's electronic health record (EHR) system using an HL7 messaging protocol or a FHIR API.
EHR System Processing: The EHR system receives the data and automatically updates the patient's health record with the new test results. These results are tagged and stored in a way that they can be easily retrieved and analyzed.
Virtual Twin Database and Mobile App Retrieval: The patient's virtual Twin Database is populated with these new data, and mobile health app is linked to the virtual twin database through secure, authenticated APIs. When new data is added to the EHR, the app is notified, and the data is synchronized with the mobile app.
Mobile App Interface and Trend Analysis: The patient opens their health app and sees a dashboard with the latest results and historical data.
   - The app displays a simple graph or trend line that shows how their results have changed over time, indicating any upward or downward trends in their biomarkers.
Alerts and Education
   - If a result is outside the normal range, the app can trigger an alert or notification, advising the patient to consult their healthcare provider.
   - The app can also provide educational content related to their results, such as diet tips for managing high glucose levels.
Provider Consultation
   - With the patient's consent, a healthcare provider can access the same data through their clinical interface, analyze trends, and make decisions about the patient's care.
   - During a consultation, the provider can pull up the patient's trends on a larger screen, discuss them with the patient, and determine if any changes in treatment are necessary.

### Example 11: examples of data collection tools

### Biology Results: Blood, Urine, cytology, Genetic

### Cardiac functional exploration: ECG and stress test

Imaging device using DICOM standard: CT (e.g., calcium scoring, CT Coronarography, Lung parenchyma), Ultrasound (e.g., abdomen, arteries), Bone density scan, Blood pressure monitoring, MRI (e.g., prostate).
Optometry device (e.g., tonometry, Auto-refractor)
Audiometry device
Medical Internet of Object: connected watch, connected Blood pressure cuff, connected blood sugar testing, connected balance, smartphone (e.g., step measurement)

### Example 12: graphical interface with interactive means

The graphical interface may be built as a "report card" that can encapsulate complex health information in a single, understandable visual. It serves not only as a snapshot of current health but also as a dynamic tool for tracking changes over time and guiding health interventions.

Presentation of the Health Risk assessment using a radar Graph (see **Figure 7****).**

The image shows a risk mapping chart comparing chronological age to physiological age across various health domains: Cardio, Diabetes, Cancer, Depression, Memory, Sleep, Vision, Audition, Mobility, etc.

The originality of this presentation lies in its visual juxtaposition of an individual's chronological age against their physiological age within the same diagram. Chronological age is a fixed measure, indicating the actual time a person has lived. In contrast, physiological age is a more flexible measure that reflects the person's health status in relation to their body's condition and functional capabilities, which can be influenced by lifestyle, genetics, and environmental factors. By overlaying these two measures on a risk assessment chart, it's possible to provide a unique perspective on an individual's health:
1. Comparative Insight: The chart allows for a direct visual comparison between the two ages, offering insight into how well a person is aging relative to their actual age.
2. Holistic Health Assessment: The inclusion of various health domains offers a comprehensive view of an individual's overall health, rather than focusing on a single aspect.
3. Health Prioritization: The chart clearly highlights areas where the physiological age exceeds the chronological age, signaling potential health risks that may require attention or intervention.
4. Personalized Health Strategies: This presentation can guide personalized health strategies, suggesting lifestyle changes or medical interventions aimed at areas where physiological age is significantly higher than chronological age.
5. Visual Engagement: The visual format is engaging and easy to understand, making it a powerful tool for communicating complex health information to individuals.

This innovative presentation of risk assessment can help individuals and healthcare providers identify potential health risks earlier and more effectively, and it facilitates a more informed discussion about health management strategies.

Interactive Graph (see **Figure 8****):**
Here's an outline of the interactive functionality such a report card offers:
Interactive Exploration of the Health Checkup: In an interactive version, clicking on or hovering over a specific domain on the spider graph could reveal more detailed information, such as specific test results, trends over time, or recommended actions to mitigate risks.

Opening a gauge chart when clicking on cardio, can be used to visually present specific health results in a straightforward and understandable way. Here's how it functions:
1. Central Needle Indicator: The needle on the gauge chart points to a value that represents the patient's condition relative to the average population. It effectively communicates whether the patient's health metric is above, below, or at the normal level.
2. Color-Coded Risk Zones: The chart is divided into color-coded zones that indicate various levels of risk or deviation from the norm. Green signifies a healthier status, yellow (e.g. between Normal (0) and +5) indicates a moderate risk, and red alerts to a high-risk condition.
3. Numerical Scale: The scale provides a quantifiable measure of how much younger or older the patient's cardiovascular health is compared to the average for their chronological age.

Positive values indicate a physiological age that is 'younger' than the average, while negative values indicate a physiological age that is 'older'.

For example, if the needle points to -12 in the green zone (e.g. between -15 and -10), it would imply that for the condition of 'cardio', the patient's physiological age is better (younger) than the average population. Conversely, if the needle points to +10 in the orange (e.g. between +5 and +15) or red zone (e.g. between +15 and +20), it suggests the patient's physiological age is significantly older for this condition, indicating a higher risk and possibly the need for medical intervention.

This gauge chart is a useful tool for quickly communicating the relative status of a patient's health for a specific condition like cardiovascular health, making it easier to understand and potentially motivating positive lifestyle changes or clinical actions. Opening a timeline chart when clicking on "trends" (see **Figure 11****):**
Opening a timeline graph that illustrates the past change in risk. As an example, the patient starts to be treated with antihypertensive 5 years before and the risk of cardiac accident decrease significantly almost immediately. For the future the timeline curve may demonstrate the potential impact of intervention (lifestyle changes or medication) on the physiological age trend of a patient. Each line represents a different scenario and how it might affect the physiological age over time, given a series of years (on the x-axis). Here's a breakdown of each series:
- A line that shows the trend of the patient's physiological age without any intervention.

This line serves as the baseline for comparison.
- A line that represents the projected trend if the patient stops smoking. Compared to the baseline, this line should ideally show a slower progression of physiological aging, reflecting the health benefits of quitting smoking.
- A line that depicts the projected trend if the patient starts exercising. Regular exercise is known to improve numerous health markers, which can lower physiological age relative to chronological age.
- A line that shows the combined effect on the physiological age trend if the patient both stops smoking and begins exercising. This scenario is expected to have the most significant positive impact, slowing the physiological aging process more than any other single intervention depicted.

The principle of this chart is to visually convey the power of lifestyle changes on an individual's health over time. By comparing these scenarios, the chart communicates that proactive health behaviors can effectively 'reverse' or slow down the aging process, as indicated by a lower physiological age. This visual tool can be used by healthcare providers to demonstrate the benefits of lifestyle interventions and motivate patients to adopt healthier habits.

Educational Tool and Personalized Health Plan: This visual report card can serve as an educational tool for patients, helping them understand the various factors contributing to their overall health and the relative importance of each. By clicking on the education bouton on a specific condition the Virtual Twin will display the recommendation for a better health in this fields, it may also guide discussions with healthcare providers about how to improve health outcomes and give access to services. The comprehensive nature of the graph supports the creation of a personalized health plan, focusing on the areas where the physiological age line extends beyond the chronological age line, indicating a need for attention.

## Claims

1. A device (100) for obtaining a trained model for prediction (31) of at least one risk score of a user for developing an abnormality with respect to a health status of the user, wherein the device (100) comprises:
- at least one input configured to receive: at least one user data (21), measured and/or obtained from said user, and at least one reference data (22), both said user data (21) and said reference data (22) being related to said abnormality;
- at least one processor configured to:
∘ generate a training dataset using said at least one user data (21) and said at least one reference data (22);
∘ obtain said trained model for prediction (31) by feeding said training dataset to a first model, wherein said trained model for prediction is configured to receive as input at least one current user data (23) and provide as output said at least one risk score of the user for developing said abnormality;
- at least one output configured to provide said trained model for prediction (31).

2. The device according to claim **1,** wherein said at least one user data (21) is obtained from at least one of a medical database, using a wearable sensor, during a clinical examination, and with a diagnostic device.

3. The device according to any of claims **1** to **2,** wherein said at least one reference data (22) is obtained from a knowledge-based database comprising established findings at least associated to said abnormality.

4. The device according to any of claims **1** to **3,** wherein said first model is an Artificial Neural Network (ANN) or a Convolutional Neural Network (CNN).

5. A device (200) for assisting the user by providing at least one health-related instruction (32) for improving a health status of the user, wherein the device (200) comprises:
- at least one input configured to receive at least one current user data (23);
- at least one processor configured to:
o calculate at least one risk score of the user for developing said abnormality using a trained model for prediction (31) obtained by the device (100) according to any of claims **1** to **4;**
o provide as input to a trained second model said at least one risk score to obtain as output said at least one health-related instruction (32), said trained second model being previously trained using a database comprising at least risk scores and associated instructions;
- at least one output configured to provide said at least one health-related instruction (32).

6. The device according to claim **5,** wherein said health-related instruction (32) is at least one of the following: a proposition to undergo a medical examination, an invitation to schedule an appointment with a healthcare professional, an advice to modify a lifestyle habit, an instruction to trigger an alert, an instruction to control a peripheral device, and an instruction to establish a communication with another user.

7. The device according to any of claims **5** to **6,** wherein a command is associated with said health-related instruction (32).

8. The device according to any of claims **5** to **7,** wherein the device (200) further comprises at least one graphical interface to display a virtual model of the user, preferably obtained using a biostatistical model.

9. The device according to claim **8,** wherein said at least one risk score is used at least to display, in the at least one graphical interface, a health status of at least one organ of the user on the virtual model of the user.

10. The device according to any of claims **5** to **9,** wherein said health-related instruction is displayed to the user on the graphical interface, and wherein the command associated with the health-related instruction (32) consists in at least one of the following:
- sending a notification to the user related to a health status of the user; and/or
- displaying a report to the user related to a health status of the user; and/or
- sending a control signal to at least one peripheral device; and/or
- sending a control signal to at least one receiving interface.

11. A computer implemented method for obtaining a trained model for prediction (31) of at least one risk score of a user for developing an abnormality with respect to a health status of the user, said method comprising:
- receiving at least one user data (21), measured and/or obtained from said user, and at least one reference data (22), both said user data (21) and said reference data (22) being related to said abnormality;
- generating a training dataset using said at least one user data (21) and said at least one reference data (22);
- obtaining said trained model for prediction by feeding said training dataset to a first model, wherein said trained model for prediction is configured to receive as input at least one current user data (23) and provide as output said at least one risk score of the user for developing said abnormality;
- providing said trained model for prediction (31).

12. A computer implemented method for assisting the user by providing at least one health-related instruction (32) for improving a health status of the user, said method for assisting comprising:
- receiving at least one current user data (23);
- calculating at least one risk score of the user for developing said abnormality using a trained model for prediction (31) obtained by the device (100) according to any of claims **1** to **4;**
- providing as input to a trained second model said at least one risk score to obtain as output said at least one health-related instruction, said trained second model being previously trained using a database comprising at least risk scores and associated instructions;
- providing said at least one health-related instruction (32).

13. A computer program product comprising instructions which, when the program is executed by a computer, cause the computer to carry out the methods of any of claims **11** to **12.**

14. A computer-readable storage medium comprising instructions which, when executed by a computer, cause the computer to carry out the methods of any of claims **11** to **12.**
